# NEW EUROPEAN PATENT SPECIFICATION

(11) **EP 1 578 477 B2**
(45) Date of publication and mention of the opposition decision: **06.05.2020**
(45) Mention of the grant of the patent: 28.09.2011
(21) Application number: 03808617.9
(22) Date of filing: 31.12.2003
(51) Int. Cl.: A61M 1/00

(54) **A dressing assembly for a closed wound or incision**
Verbandsystem für eine geschlossene Wunde oder Schnittwunde
Système de pansement pour une plaie fermée ou une incision

(30) Priority: 31.12.2002 US 334766; 08.04.2003 US 409225
(43) Date of publication of application: 28.09.2005
(73) Proprietor: KCI Licensing, Inc., San Antonio, TX 78265 (US)
(72) Inventor: BUBB, Stephen, K., Kansas City, MO 64113 (US); ZAMIEROWSKI, David, S., Overland Park, KS 66209 (US)
(74) Representative: Simmons & Simmons
(86) International application number: PCT/US2003/041667
(87) International publication number: WO 2004/060148

(56) References cited:
- WO-A-01/85248
- WO-A-01/89431
- WO-A1-95/17146
- US-A- 4 250 882
- US-A- 4 899 762
- US-A- 5 291 887
- US-A- 6 071 267
- US-B1- 6 174 306
- WEBB, l.: "NEW TECHNIQUES IN WOUND MANAGEMENT: VACUUM-ASSISTED WOUND CLOSURE", Perspectives on Modern Orthopaedics, vol. 10, no. 5, 1 October 2002 (2002-10-01), pages 303-311,

## Description

### BACKGROUND OF THE INVENTION

### 1. Field of the Invention

The present invention relates generally to medical devices for treating closed wounds and incisions, and in particular to a system for draining and/or irrigating tissue separations, such as surgical incisions, and for compressing and stabilizing a dissected or traumatized field with ambient air pressure created by an external patient interface component and a vacuum source.

### 2. Description of the Related Art

Tissue separations can result from surgical procedures and other causes, such as traumatic and chronic wounds. Various medical procedures are employed to close tissue separations. An important consideration relates to securing separate tissue portions together in order to promote closure and healing. Incisions and wounds can be closed with sutures, staples and other medical closure devices. The "first intention" (primary intention healing) in surgery is to "close" the incision. For load-bearing tissues, such as bone, fascia, and muscle, this requires substantial material, be it suture material, staples, or plates and screws. For the wound to be "closed," the epithelial layer must seal. To accomplish this, the "load bearing" areas of the cutaneous and subcutaneous layers (i.e., the deep dermal elastic layer and the superficial fascia or fibrous layers of the adipose tissue, respectively) must also at least be held in approximation long enough for collagen deposition to take place to unite the separated parts.

Other important considerations include controlling bleeding, reducing scarring, eliminating the potential of hematoma, seroma, and "dead-space" formation and managing pain. Dead space problems are more apt to occur in the subcutaneous closure. Relatively shallow incisions can normally be closed with surface-applied closure techniques, such as sutures, staples, glues and adhesive tape strips. However, deeper incisions may well require not only skin surface closure, but also time-consuming placement of multiple layers of sutures in the load-bearing planes.

Infection prevention is another important consideration. Localized treatments include various antibiotics and dressings, which control or prevent bacteria at the incision or wound site. Infections can also be treated and controlled systemically with suitable antibiotics and other pharmacologics.

Other tissue-separation treatment objectives include minimizing the traumatic and scarring effects of surgery and minimizing edema. Accordingly, various closure techniques, postoperative procedures and pharmacologics are used to reduce postoperative swelling, bleeding, seroma, infection and other undesirable, postoperative side effects. Because separated tissue considerations are so prevalent in the medical field, including most surgeries, effective, expedient, infection-free and aesthetic tissue closure is highly desirable from the standpoint of both patients and health-care practitioners. The system, interface and method of the present invention can thus be widely practiced and potentially provide widespread benefits to many patients.

Fluid control considerations are typically involved in treating tissue separations. For example, subcutaneous bleeding occurs at the fascia and muscle layers in surgical incisions. Accordingly, deep drain tubes are commonly installed for the purpose of draining such incisions. Autotransfusion has experienced increasing popularity in recent years as equipment and techniques for reinfusing patients' whole blood have advanced considerably. Such procedures have the advantage of reducing dependence on blood donations and their inherent risks. Serous fluids are also typically exuded from incision and wound sites and require drainage and disposal. Fresh incisions and wounds typically exude blood and other fluids at the patient's skin surface for several days during initial healing, particularly along the stitch and staple lines along which the separated tissue portions are closed.

Another area of fluid control relates to irrigation. Various irrigants are supplied to separated tissue areas for countering infection, anesthetizing, introducing growth factors and otherwise promoting healing. An effective fluid control system preferably accommodates both draining and irrigating functions sequentially or simultaneously.

Common orthopedic surgical procedures include total joint replacements (TJRs) of the hip, knee, elbow, shoulder, foot and other joints. The resulting tissue separations are often subjected to flexure and movement associated with the articulation of the replacement joints. Although the joints can be immobilized as a treatment option, atrophy and stiffness tend to set in and prolong the rehabilitation period. A better option is to restore joint functions as soon as possible. Thus, an important objective of orthopedic surgery relates to promptly restoring to patients the maximum use of their limbs with maximum ranges of movement.

Similar considerations arise in connection with various other medical procedures. For example, arthrotomy, reconstructive and cosmetic procedures, including flaps and scar revisions, also require tissue closures and are often subjected to movement and stretching. Other examples include incisions and wounds in areas of thick or unstable subcutaneous tissue, where splinting of skin and subcutaneous tissue might reduce dehiscence of deep sutures. The demands of mobilizing the extremity and the entire patient conflict with the restrictions of currently available methods of external compression and tissue stabilization. For example, various types of bandage wraps and compressive hosiery are commonly used for these purposes, but none provides the advantages and benefits of the present invention

The aforementioned procedures, as well as a number of other applications discussed below, can benefit from a tissue-closure treatment system and method with a surface-applied patient interface for fluid control and external compression.

Postoperative fluid drainage can be accomplished with various combinations of tubes, sponges, and porous materials adapted for gathering and draining bodily fluids. The prior art includes technologies and methodologies for assisting drainage. For example, the Zamierowski U.S. Patents No. 4,969,880; No. 5,100,396; No. 5,261,893; No. 5,527,293; and No. 6,071,267 disclose the use of pressure gradients, i.e., vacuum and positive pressure, to assist with fluid drainage from wounds, including surgical incision sites. Such pressure gradients can be established by applying porous sponge material either internally or externally to a wound, covering same with a permeable, semi-permeable, or impervious membrane, and connecting a suction vacuum source thereto. Fluid drawn from the patient is collected for disposal. Such fluid control methodologies have been shown to achieve significant improvements in patient healing. Another aspect of fluid management, postoperative and otherwise, relates to the application of fluids to wound sites for purposes of irrigation, infection control, pain control, growth factor application, etc. Wound drainage devices are also used to achieve fixation and immobility of the tissues, thus aiding healing and closure. This can be accomplished by both internal closed wound drainage and external, open-wound vacuum devices applied to the wound surface. Fixation of tissues in apposition can also be achieved by bolus tie-over dressings (Stent dressings), taping, strapping and (contact) casting.

A prior art wound dressing is disclosed in International patent application, publication no. WO 01/85248. This document teaches a wound dressing (referenced 10 in the patent drawings thereof) having a dual layer of foam (12, 36), the lower foam (36) being covered by an elastomeric film (38) having a plurality of placed holes (34) and the upper layer of foam (12) being covered by an adhesive elastomeric sheet (14) which adheres the dressing to a patient.

Heretofore there has not been available a dressing assembly with the advantages and features of the present invention.

### SUMMARY OF THE INVENTION

According to the present invention, we provide a dressing assembly as set forth in claim 1 appended hereto. Further features of the present invention are set out in claims 2 and 3 appended hereto.

In the practice of the present invention, a system is provided for enhancing closure of separated tissue portions using a surface-applied patient interface. Subsurface drainage, irrigation and autotransfusion components can optionally be used in conjunction with the surface-applied, external interface. The external interface can be advantageously placed over a stitch or staple line and includes a primary transfer component comprising a strip of porous material, such as rayon, applied directly to the patient for wicking or transferring fluid to a secondary transfer component comprising a sponge or foam material. An underdrape is placed between the transfer elements for passing fluid therebetween through an underdrape opening, such as a slot. An overdrape is placed over the secondary transfer component and the surrounding skin surface. The patient interface is connected to a negative pressure source, such as a vacuum assisted closure device, wall suction or a mechanical suction pump. A manual control embodiment utilizes a finite capacity fluid reservoir with a shut-off valve for discontinuing drainage when a predetermined amount of fluid is collected. An automatic control embodiment utilizes a microprocessor, which is adapted for programming to respond to various inputs in controlling the operation of the negative pressure source. A closed wound or incision treatment method of the present invention involves three phases of fluid control activity, which correspond to different stages of the healing process. In a first phase active drainage is handled. In a second phase components can be independently or sequentially disengaged. In a third phase the secondary transfer component can optionally be left in place for protection and to aid in evacuating any residual fluid from the suture/staple line through the primary transfer component.

In other embodiments of the invention, components of the dressing system can be premanufactured for efficient application. A foam piece can be provided with a full or partial rayon cover and a close-fitting overdrape. An access panel with a reclosable seal strip can be installed on the overdrape for access to the foam pieces and the wound area. A premanufactured external dressing can be provided with a sheath receiving a foam piece, which is accessible through a reclosable seal strip for replacement or reorientation. Treatment area access is also provided through the seal strip.

### BRIEF DESCRIPTION OF THE DRAWINGS

The drawings constitute a part of this specification and include exemplary embodiments of the present invention and illustrate various objects and features thereof.
Fig. 1 is a schematic, block diagram of a tissue closure treatment and system embodying the present invention.
Fig. 2 is a perspective view of an incision tissue separation with a deep drain tube installed.
Fig. 3 is a perspective view thereof, showing the separated tissue sutured together at the skin.
Fig. 4 is a perspective view thereof, showing the separated tissue sutured together at the deep dermal layer below the skin surface.
Fig. 5 is a perspective view thereof, showing a rayon strip primary fluid transfer component (FTC.1) and an underdrape being placed on the stitch line.
Fig. 6 is a perspective view thereof, showing FTC.1 and the underdrape in place on the stitch line.
Fig. 7 is a perspective view thereof, showing a secondary fluid transfer component (FTC.2) in place.
Fig. 8 is a perspective view thereof, showing an overdrape in place.
Fig. 9 is a perspective view thereof, showing a connecting fluid transfer component (FTC.3) in place for connecting the system to a negative pressure source.
Fig. 10 is a cross-sectional view thereof, taken generally along line 10-10 in Fig. 9 and particularly showing FTC.3.
Fig. 11a is a perspective view thereof, showing FTC.3 removed and the overdrape scored for ventilation.
Fig. 11b is a perspective view thereof, showing the patient interface removed along a perforated tear line in the underdrape and a slit line in the overdrape.
Fig. 11c is a perspective view of a patient interface adapted for prepackaging, application to a patient and connection to a negative pressure source.
Figs. 12a-d show alternative embodiment elbow connecting devices FTC.3a-d respectively.
Figs. 12e,f show a modified FTC.2a with removable wedges to facilitate articulation, such as flexure of a patient joint.
Figs.12g,h show alternative embodiment external patient interface assemblies.
Figs. 13a-c comprise a flowchart showing a tissue closure treatment method embodying the present invention.
Fig. 14 is a schematic, block diagram of an automated tissue closure treatment system comprising an alternative embodiment of the present invention.
Fig. 15 is a cross-sectional view of the alternative embodiment automated tissue closure treatment system.
Fig. 16 is a partial flowchart of an alternative embodiment automated tissue closure treatment method embodying the present invention.
Fig. 17 is a fragmentary, perspective view of a tissue closure treatment system comprising an alternative embodiment of the present invention, with a reclosable access panel.
Fig. 18 is a perspective view of the reclosable access panel.
Fig 19 is a cross-sectional view of the tissue closure treatment system, taken generally along line 19-19 in Fig 18.
Fig 20 is an enlarged, cross-sectional view of the tissue closure system, particularly showing a reclosable seal strip thereof.
Fig. 21 is a perspective view of the tissue closure system, showing the seal strip open.
Fig 22 is a perspective view of the tissue closure system, showing the seal strip open and a foam piece removed.
Fig 23 is a cross-sectional view of an external dressing assembly, which comprises an alternative embodiment of the present invention.
Fig. 24 is a cross-sectional view of an alternative embodiment tissue closure system with internal and external foam pieces.
Fig. 25 is a cross-sectional view of the system shown in Fig 24, showing the progressive healing of tissue in the wound.
Fig. 26 is a cross-sectional view of the system shown in Fig 24, showing the reepithelialization of the wound.
Fig 27 is a cross-sectional view of a foam piece partially enclosed in rayon.
Fig. 28 is a cross-sectional view of an alternative embodiment tissue closure system, with an external foam piece and an internal foam piece assembly.
Fig. 29 is a cross-sectional view thereof, shown partially collapsed under ambient atmospheric pressure.
Fig. 30 is a perspective view of an alternative construction dressing with a reclosable seal strip and fluid access ports.
Fig. 31 is a perspective view of the underside of the dressing, showing a middle backing strip being removed.
Fig. 32 is a perspective view of the dressing, showing side backing strips being removed.
Fig. 33 is a perspective view of the dressing, shown with a squeeze bulb evacuator attached to a fluid port thereof.
Fig 34 is a perspective view of the dressing, shown partially-collapsed under atmospheric pressure.
Fig. 35 is a perspective view of the dressing, shown with the seal strip open.
Fig. 36 is a perspective view of the dressing, shown with the foam piece removed.
Fig. 37 is a cross-sectional view of a foam piece fully-enclosed in rayon.
Fig 38 is a perspective view of an alternative embodiment dressing with a separate liner and foam piece.
Fig. 39 is a perspective view of the dressing, shown with the foam piece for moved.
Fig. 40 is a perspective view of the dressing, shown with the liner removed.
Fig. 41 is a cross-sectional view of an alternative embodiment dressing with a sheath bottom panel comprising a wicking material.
Fig. 42 is a cross-sectional view of an alternative embodiment dressing system with a covered foam-core transfer element.
Fig. 43 is a cross-sectional view thereof, showing the dressing compressed under pressure.
Fig. 44 is a top plan view thereof.
Fig. 45 is a cross-sectional view thereof, showing the dressing configuration prior to application to a patient and taken generally along line 45-45 in Fig. 44.
Fig. 46 is a top plan view of an application involving multiple dressings covering an elongated tissue separation, such as a surgical incision.

### DETAILED DESCRIPTION OF THE PREFERRED EMBODIMENT

### I. Introduction and Environment

As required, detailed embodiments of the present invention are disclosed herein; however, it is to be understood that the disclosed embodiments are merely exemplary of the invention, which may be embodied in various forms. Therefore, specific structural and functional details disclosed herein are not to be interpreted as limiting, but merely as a basis for the claims and as a representative basis for teaching one skilled in the art to variously employ the present invention in virtually any appropriately detailed structure.

### II. Tissue Closure System 2

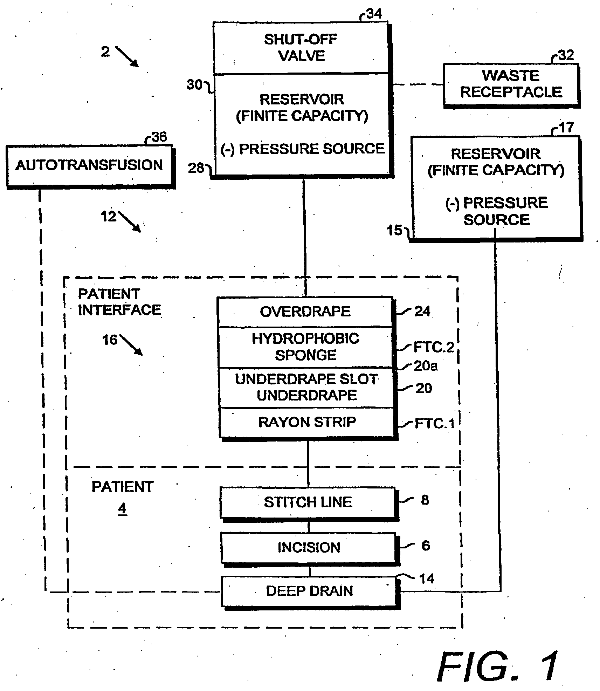

Referring to the drawings in more detail, the reference numeral 2 generally designates a tissue closure treatment system embodying the present invention. As shown in Fig. 1, the system 2 is adapted for use on a patient 4 with an incision or wound 6, which can be closed by a stitch line 8 consisting of sutures 10, staples or other suitable medical fasteners.

A patient interface 12 consists of an optional deep drain 14 connected to a deep drain negative pressure source 15 associated with a deep drainage reservoir 17 and an external patient interface 16 including a primary fluid transfer component FTC.1 comprising a strip of rayon or other suitable porous material, an underdrape 20 generally covering FTC.1 and including a slot 20a, a secondary fluid transfer component FTC.2 comprising a hydrophobic sponge and an overdrape 24.

A fluid handling subsystem 26 includes the deep drain negative pressure source 15 and a surface drain negative pressure source 28, which can be combined for applications where a common negative pressure source and a collection receptacle are preferred. The negative pressure sources 15, 28 can operate either manually or under power. Examples of both types are well-known in the medical art. For example, a manually operable portable vacuum source (MOPVS) is shown in U.S. Patent No. 3,115,138. The MOPVS is available from Zimmer, Inc. of Dover, Ohio under the trademark HEMOVAC®. Bulb-type actuators, such as that shown in U.S. Patent N0.4,828,546 and available from Surgidyne, Inc. of Eden Prairie, Minnesota, can be used on smaller wounds, for shorter durations or in multiples. Moreover, power-actuated vacuum can be provided by vacuum assisted closure equipment available under the trademark THE VAC® from Kinetic Concepts, Inc. of San Antonio, Texas. Still further, many health-care facilities, particularly hospitals and clinics, are equipped with suction systems with sources of suction available at wall-mounted outlets.

A finite capacity reservoir 30 is fluidically connected to the negative pressure source 28 and is adapted to discharge to a waste receptacle 32. A shut-off valve 34 is associated with the reservoir 30 and is adapted to automatically discontinue drainage when the reservoir 30 is filled to a predetermined volume.

An optional autotransfusion subsystem 36 can be connected to the deep drain 14 and is adapted for reinfusing the patient 4 with his or her own blood. U.S. Patent No. 5,785,700 discloses such an autotransfusion system with a portable detachable vacuum source, which is available from Zimmer, Inc.

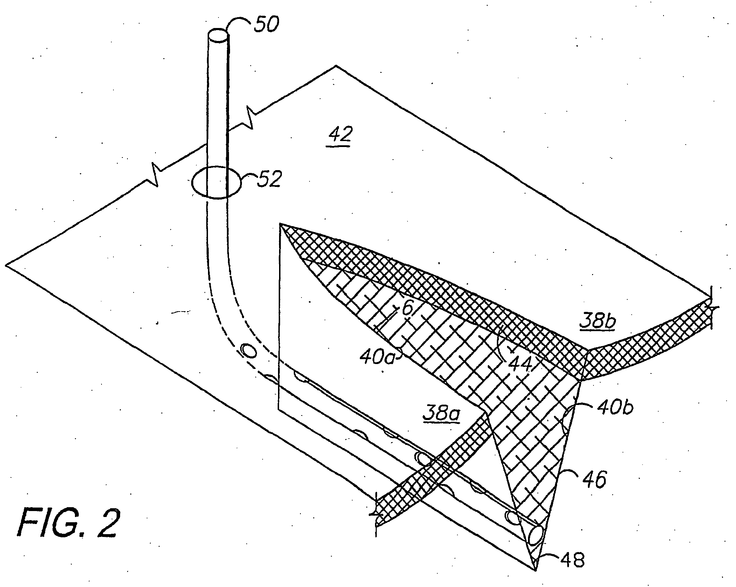

Fig. 2 shows an incision 6 forming first and second separated tissue portions 38a,b with incision edges 40a,b. The incision 6 extends from and is open at the skin 42, through the deep dermal layer 44 and the subcutaneous layer 46, to approximately the fascia 48. A deep drain tube 50 is placed in a lower part of the incision 6 and penetrates the skin 42 at an opening 52..

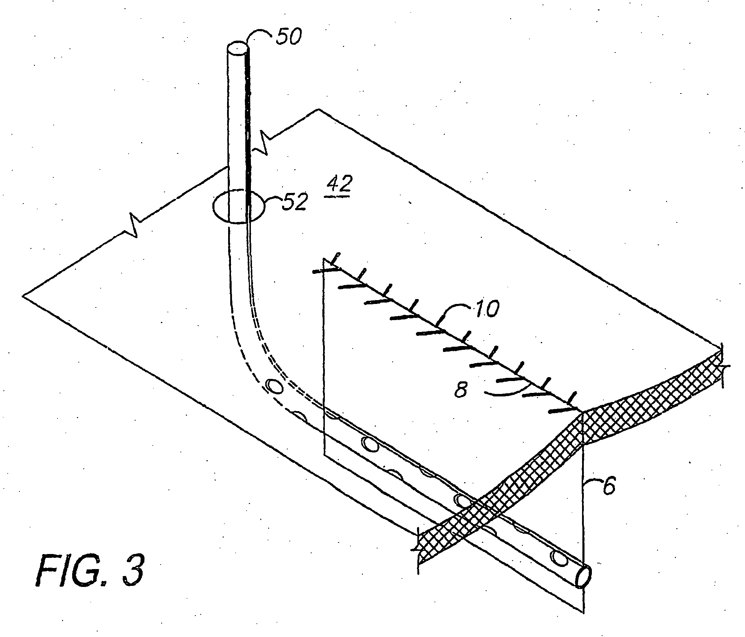

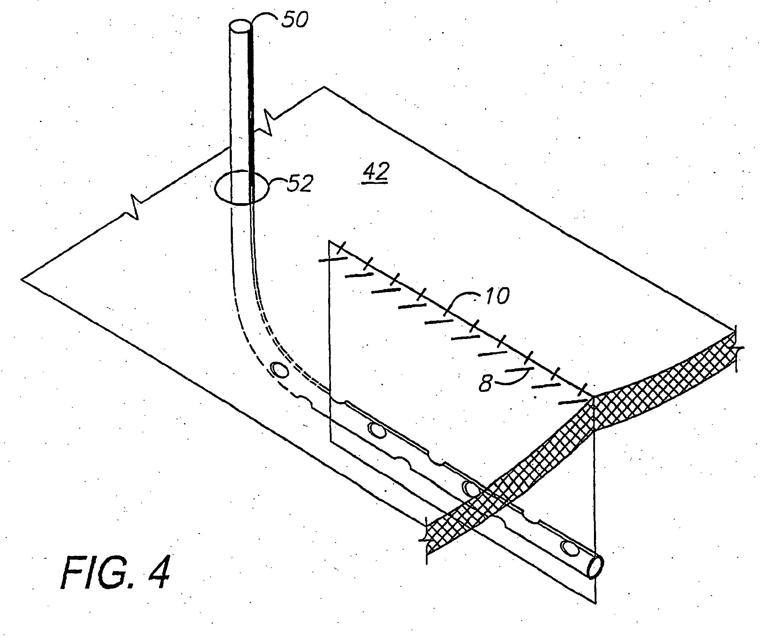

Fig. 3 shows the incision edges 40a,b secured together by sutures 54 forming a stitch line 56 at the skin surface 42. As an alternative to sutures 54, various other medical fasteners, such as staples, can be used. Fig. 4 shows sutures 55 placed in the deep dermal layer 44 below the skin surface 42.

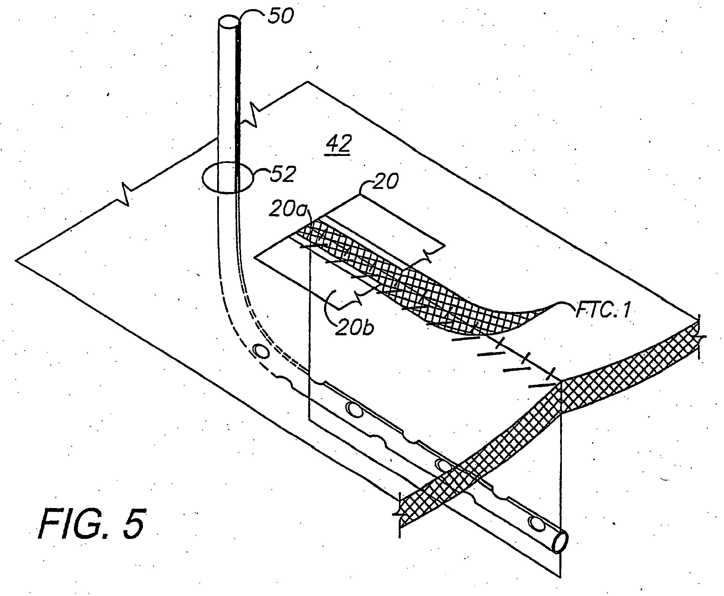

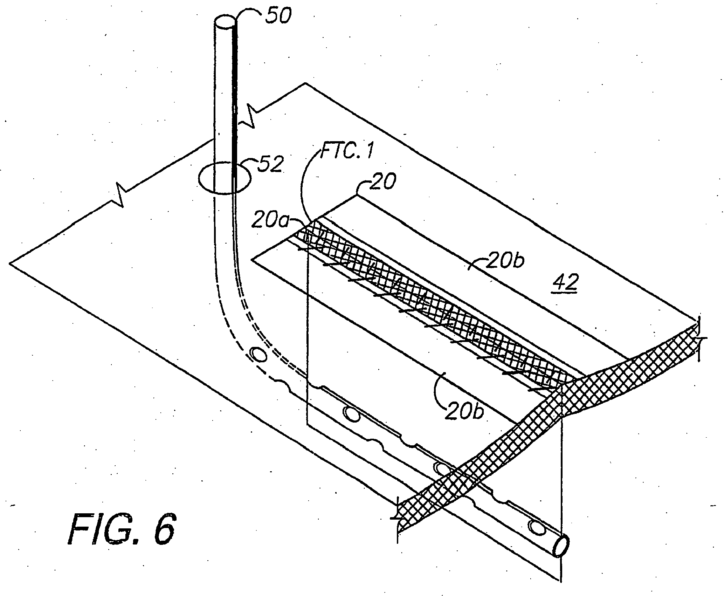

Fig. 5 shows application of FTC.1 on top of the stitch line 8. FTC.1 preferably comprises a suitable porous wicking material, such as rayon, which is well-suited for wicking the fluid that exudes along the stitch line 8. Rayon also tends to dry relatively quickly, and thus efficiently transfers fluid therethrough. The underdrape 20 is placed over FTC.1 and the adjacent skin surface 42. Its slot 20a is generally centered along the centerline of FTC.1 and directly above the stitch line 8. FTC.1 and the underdrape 20 can be preassembled in a roll or some other suitable configuration adapted to facilitate placement on the stitch line 8 in any desired length. Fig. 6 shows FTC.1 and the underdrape 20 in place.

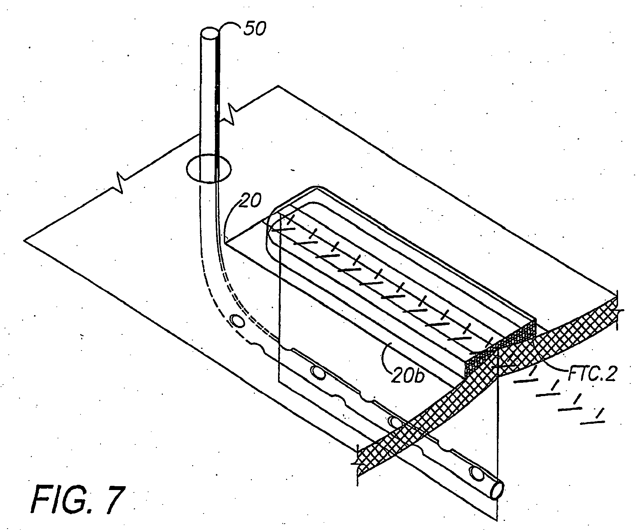

The secondary fluid transfer component FTC.2 is shown installed in Fig. 7. It preferably comprises a suitable hydrophobic foam material, such as polyurethane ether (PUE), which comprises a reticulated, lattice-like (foam) material capable of being collapsed by vacuum force (negative pressure) in order to exert positive "shrink-wrap" type compression on skin surface and still maintain channels that allow passage of fluid. As shown, its footprint is slightly smaller than that of the underdrape 20, thus providing an underdrape margin 20b. The wicking layer of FTC.1 can, as an alternative, be sized equal to or almost equal to the footprint of FTC.2. This configuration lends itself to prefabrication as an individual, pre-assembled pad that can be employed by simply removing a releasing layer backing from an adhesive lined underdrape. This configuration also lends itself to easy total removal and replacement of the central part of the assembly without removing drape already adhered to skin if removal and replacement is the desired clinical option rather then staged removal or prolonged single application.

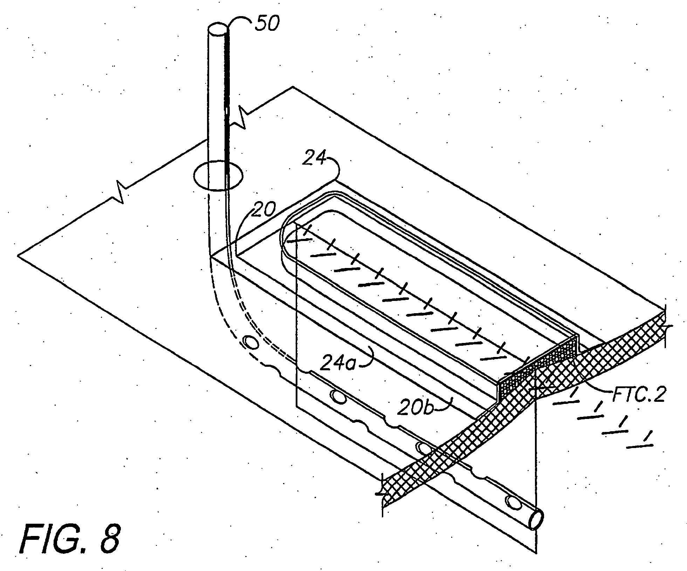

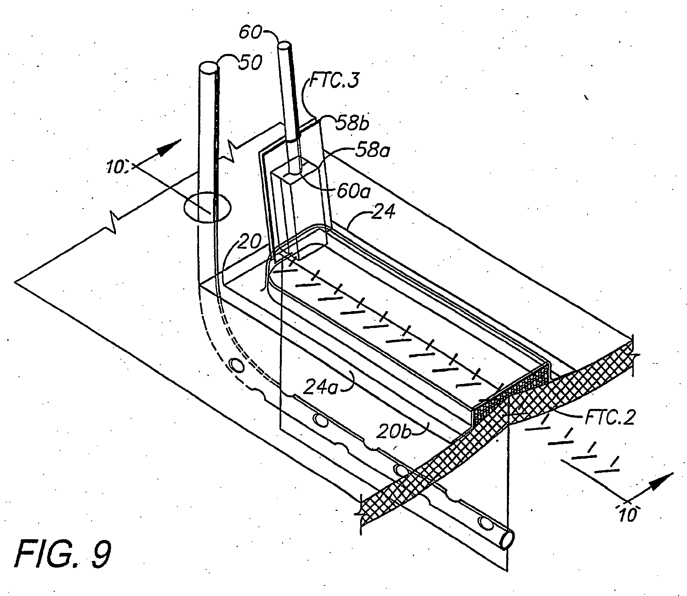

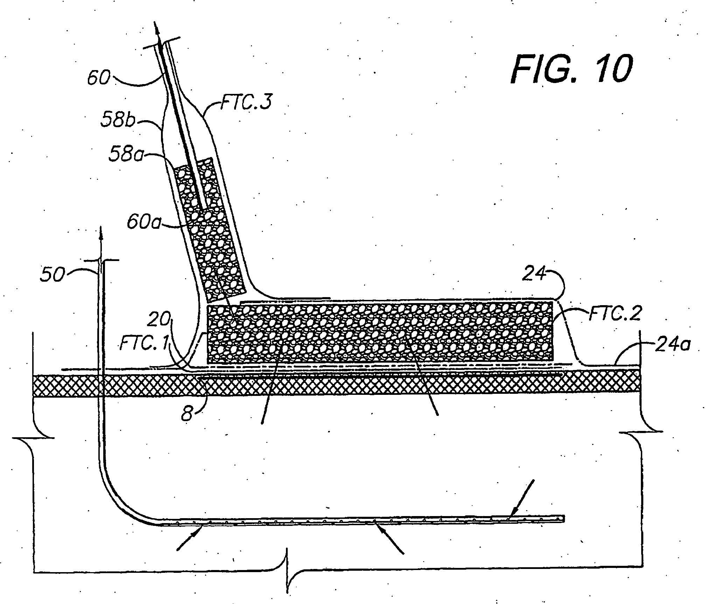

Fig. 8 shows the overdrape 24 applied over FTC.2 and the underdrape 20, with a margin 24a extending beyond the underdrape margin 22b and contacting the patient's skin surface (dermis) 42. Figs. 9 and 10 show a patch connector 58 mounted on FTC.2 and comprising a hydrophobic foam (PUE) material core 58a sandwiched between drape layers 58b. A vacuum drain tube 60 includes an inlet end 60a embedded in the foam core 58a and extends between the drape layers 58b to an outlet end 60b connected to the surface drainage negative pressure source 28.

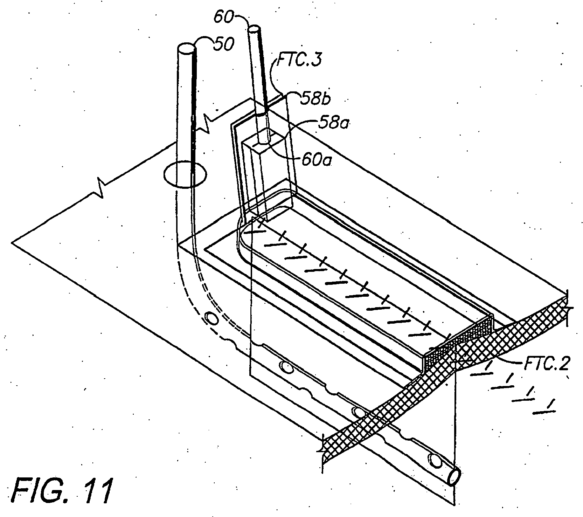

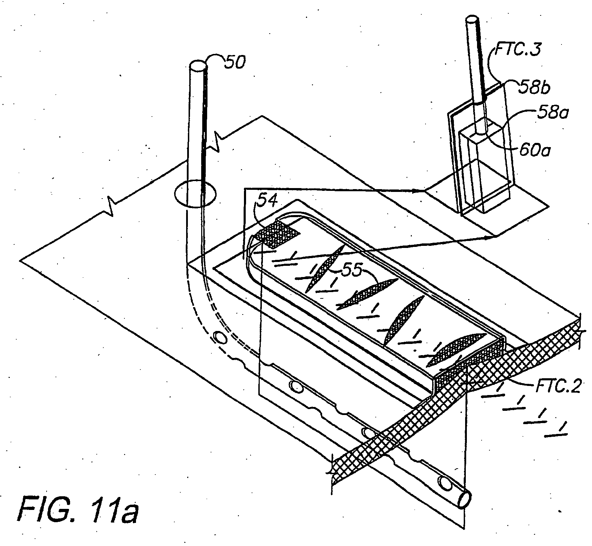

Fig. 11a shows FTC.3 removed, e.g. by cutting away portions of the overdrape 24 to provide an overdrape opening 54. In addition, the overdrape 24 can be slit at 55 to further ventilate FTC.2. Draining FTC.2 under negative pressure, and further drying it with air circulation (Fig. 11a) can provide significant healing advantages by reducing the growth of various microbes requiring moist environments in FTC.2. Such microbes and various toxins produced thereby can thus be evaporated, neutralized and otherwise prevented from reentering the patient. Microbe control can also be accomplished by introducing antiseptics in and irrigating various components of the patient interface 12, including the drapes 20, 24; FTC.1; FTC.2; and FTC.3.

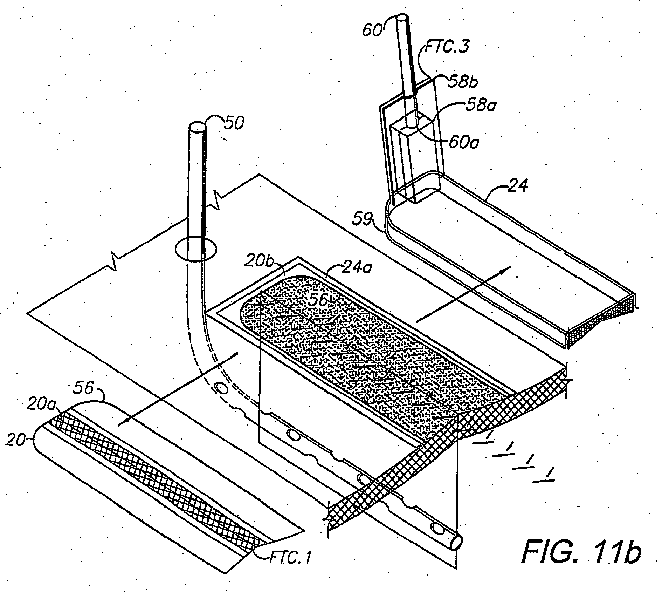

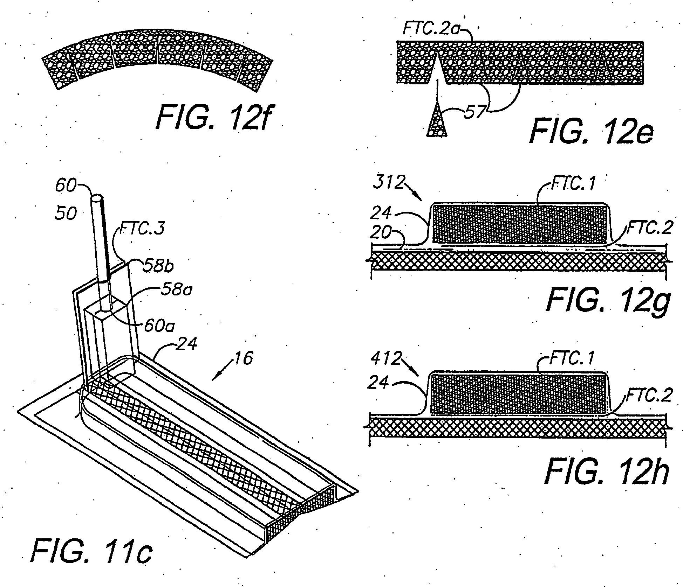

Fig. 11b shows the patient interface 12 removed along underdrape perforated tear lines 56 and slit lines 59 in overdrape 24. It will be appreciated that substantially the entire patient interface 12, except for underdrape and overdrape margins 20b, 24a can thus be removed to provide access to the stitch line 8 and the dermis 42 for visual inspection, evaluation, cleaning, stitch removal, dressing change (e.g., with prepackaged patient interface 12a as shown in Fig. 11c), consideration of further treatment options, etc. For example, the overdrape 24 can be slit to around the perimeter or footprint of FTC.2 to permit removing the same. Preferably FTC.2 is easily releasable from the underdrape 20 and FTC.1 whereby FTC.2 can be grasped and lifted upwardly to facilitate running a scalpel through the overdrape 24 and into a separation between the underside of FTC.2 and the underdrape 20. The FTC.1 can then optionally be removed by tearing the underdrape 20 along its tear lines 56 and removing same as shown in Fig. 11b.

Fig. 11c shows a prepackaged patient interface 12a adapted for initial or "dressing change" application. Optionally, the rayon strip FTC.1 can have the same configuration or "footprint" as the foam sponge FTC.2, thus eliminating the underdrape 20. The prepackaged patient interface 12a can be sterilely packaged to facilitate placement directly on a stitch line 8. Alternatively, the patient interface components can be prepackaged individually or in suitable groups comprising subassemblies of the complete patient interface 12. For example, the underdrape/FTC.1 and the overdrape/FTC.2 subassemblies respectively can be prepackaged individually. Various sizes and component configurations of the patient interface can be prepackaged for application as indicated by particular patient conditions. Preferably, certain sizes and configurations would tend to be relatively "universal" and thus applicable to particular medical procedures, such as TJRs, whereby patient interface inventory can be simplified. Alternatively, the individual components can be assembled in various sizes and configurations for "custom" applications.

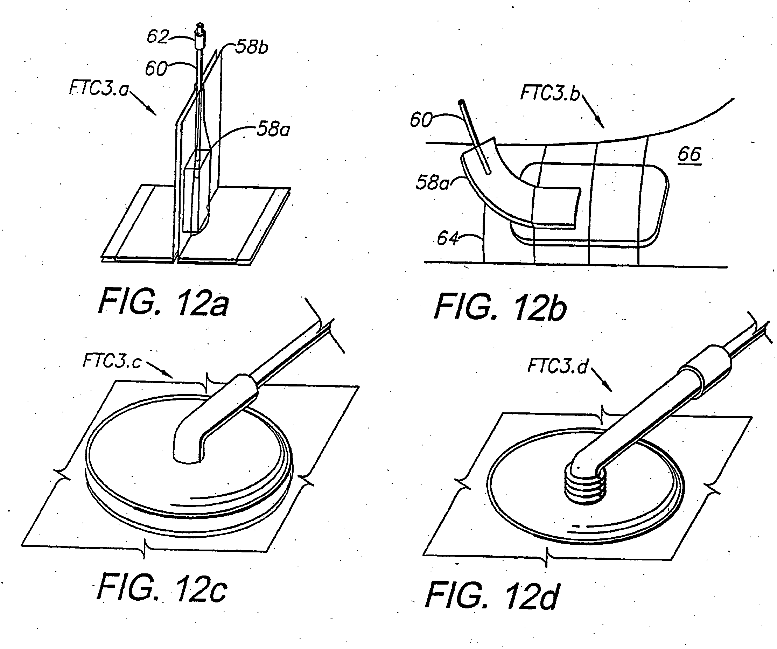

Figs 12a-d show alternative connecting fluid transfer components FTC.3a-d for connecting FTC.2 to the surface drainage negative pressure source 28. FTC.3a (Fig. 12a) shows a patch connector with a similar construction to FTC.3 and adapted for placement at any location on the overdrape 24. FTC.3a is provided with a Leur lock connector 62. FTC.3b (Fig. 12b) comprises a strip of hydrophobic (PUE) foam material partially covered by an overdrape 64, which can be configured as a wrap around a patient's limb or extremity 66. FTC.3c (Fig.12c) is an elbow-type connector. FTC.3d (Fig. 12d) is a bellows-type elbow connector, which is adapted to accommodate deflection of the vacuum drain tube 60.

Figs.12e,f show an alternative construction of FTC.2a with multiple, removable wedges 57 formed therein and adapted for accommodating articulation, such as joint flexure. The flexibility of FTC.2a can thus be considerably enhanced for purposes of patient comfort, mobility and flexibility. Such wedges can extend transversely and/or longitudinally with respect to FTC.2a. FTC.2a functions in a similar manner with and without the wedges 57 in place or removed.

Fig. 12g shows a modified patient interface 312 with the underdrape 20 placed below FTC.1. This configuration permits removing FTC.1 without disturbing the underdrape 20. Fig. 12h shows a further modified patient interface 412 with FTC.1 having the same configuration or footprint as FTC.2, whereby they can be fabricated and bonded together. In this configuration the underdrape 20 can be omitted.

### III. Treatment Method

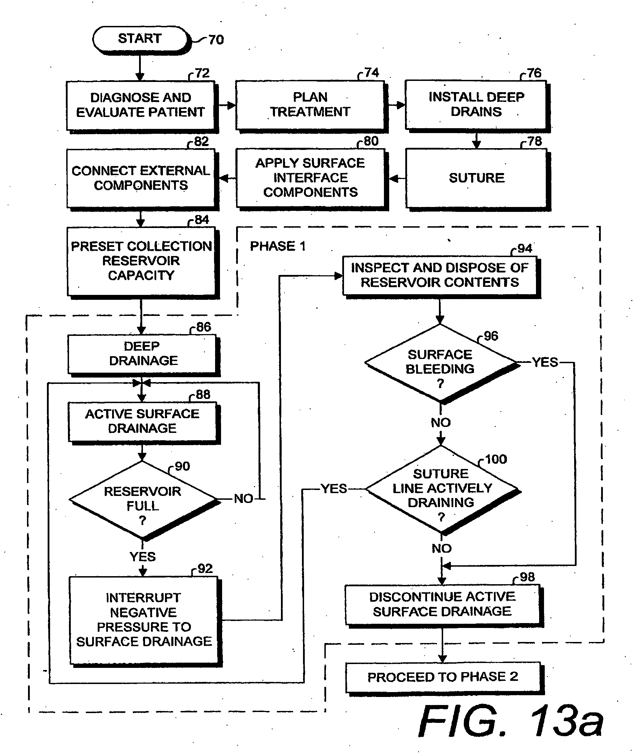

Figs.13a-c comprise a flowchart for a method not forming part of the present invention. From start 70 the method proceeds to patient diagnosis and evaluation at 72 and treatment plan at 74. Deep drains 14 are installed at 76 as necessary, and the incision is sutured at 78. Surface interface components 12 are applied at 80 and connected to the external components (i.e., negative pressure sources 15, 28) at 82. The collection reservoir capacity is preset at 84 based on such factors as nature of wound/incision, blood flow, etc.

### Phase 1

Deep drainage occurs at 86 and active surface drainage occurs at 88, both being influenced by the negative pressure sources 15, 28. The negative pressure source 28 causes the PUE foam FTC.2 to partially collapse, which correspondingly draws down the overdrape 24 and exerts a positive, compressive force on the closed wound or incision 6. In the closed environment of the patient interface 12, such force is effectively limited to ambient atmosphere. This limiting control feature protects the patient from excessive force exerted by the patient interface 12. The steady force of up to one atmosphere applied across the closed wound or incision 6 functions similarly to a splint or plaster cast in controlling edema and promoting healing.

A "Reservoir Full" condition is detected at 90 and branches to an interrupt of the surface drainage negative pressure at 92, after which the reservoir contents are inspected and disposed of at 94. If surface bleeding is detected by visual inspection at decision box 96, the method branches to a "Discontinue Active Surface Drainage" step at 98. If the suture line is actively draining at decision box 100, the method loops to the active surface drainage step 88 and continues, otherwise active surface drainage discontinues at 98, i.e. when the wound/incision is neither bleeding nor exuding fluids.

Phase 1 is generally characterized by deep drainage (interactive or passive) and active surface drainage under the influence of manual or powered suction. The normal duration is approximately two to three days, during which time post-operative or post-trauma swelling normally reaches its maximum and begins to recede.

### Phase 2

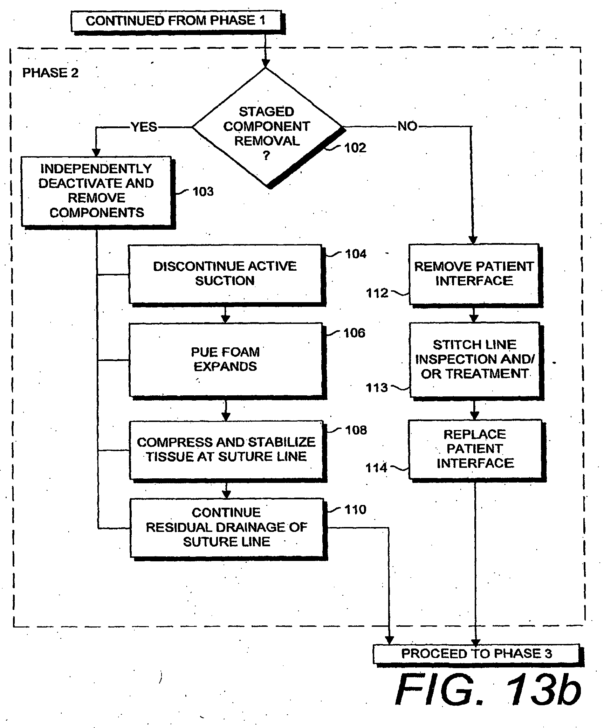

Fig. 13b shows Phase 2 commencing with a "Staged Component Removal?" decision box 102. An affirmative decision leads to independently deactivating and removing components at 103, including discontinuing active suction at 104, which transforms the hydrophobic PUE foam (FTC.2) internal pressure from negative to positive and allows the collapsed FTC.2 to reexpand at 106, potentially increasing surface composite pressure from ambient to positive. Preferably this transition occurs without applying undue pressure to the surface from the decompressed, expanding FTC.2. During Phase 1, negative pressure (i.e., suction/vacuum) tends to compress FTC.2 and correspondingly contracts the overdrape 24, adding to the compression exerted by FTC.2. When the application of negative pressure discontinues, either manually or automatically, FTC.2 re-expands against the constraints of the overdrape 24, and in an equal and opposite reaction presses against the skin 42, particularly along the stitch line 8. FTC.2 can thus automatically transform from ambient to positive pressure simply by discontinuing the application of the vacuum source.

The positive pressure exerted on the skin 42 continues to compress and stabilize tissue along the suture line 8 (step 108) in order to reduce swelling and cooperates with the operation of FTC.1 and FTC.2 to continue drainage by evaporation at the suture line 8 at step 110. A negative determination at decision box 102 leads to interface removal at 112 and, unless treatment is to be terminated, stitch line inspection and treatment at 113 and interface replacement at 114, which can involve all or part of the patient interface 12. The method then proceeds to Phase 3. Phase 3

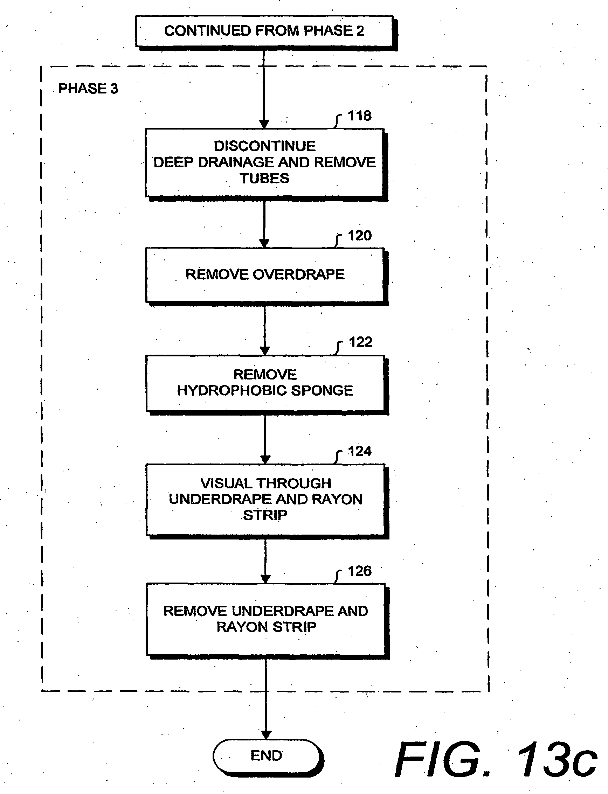

Fig. 13c shows Phase 3 of the treatment method wherein deep drainage is discontinued and the tube(s) is removed at 118. The overdrape 24 and FTC.2 are removed at 120, 122 respectively. The underdrape 20 and FTC.1 are preferably configured to permit visual inspection of the suture line 8 therethrough at 124. When the suture line 8 has closed sufficiently, the underdrape 20 and FTC.1 are removed at 126 and the treatment ends at 128. Alternatively and if indicated by the patient's condition, all or part of the interface 12 can be replaced in Phase 3 and treatment continued.

### IV. Alternative Embodiment Tissue Closure System 202

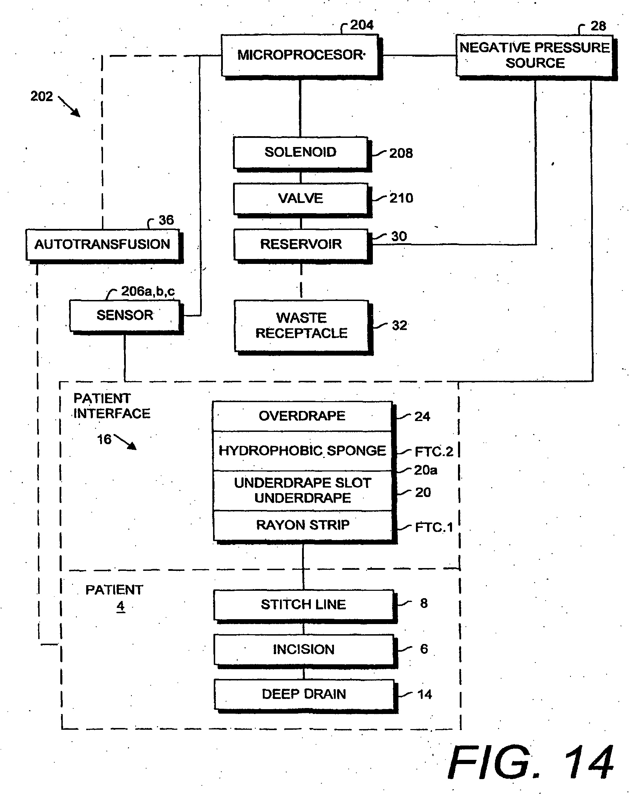

Fig. 14 schematically shows a tissue closure system 202 comprising an alternative embodiment of the present intention, which includes a microprocessor or controller 204, which can be connected to one or more sensors 206 coupled to the patient interface 12 for sensing various conditions associated with the patient 4. The microprocessor 204 can be programmed to operate a solenoid 208 coupled to a valve 210 associated with the reservoir 30 and controlling fluid flow induced by a negative pressure source 228 through its connection to the patient interface 12.

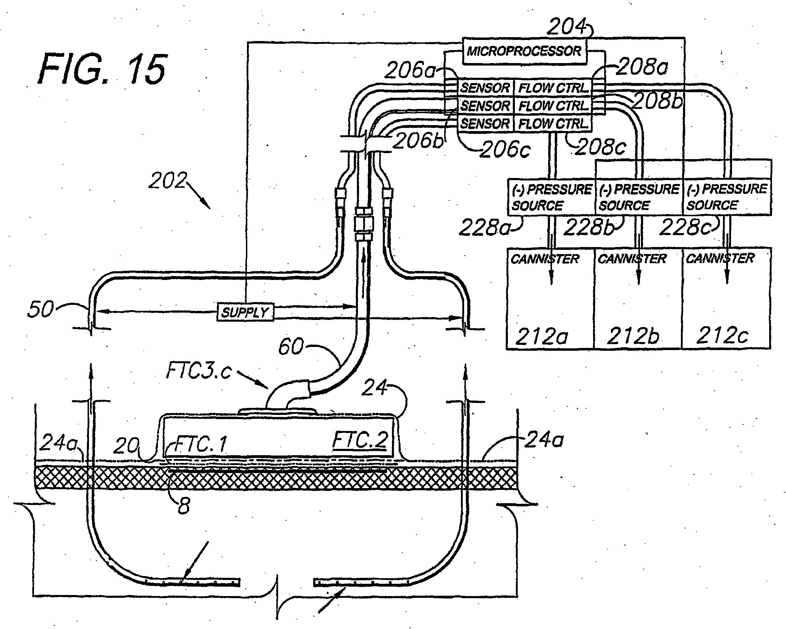

Fig. 15 shows the tissue closure system 202 with the microprocessor 204 connected to multiple sensors 206a,b,c each of which is associated with a flow control component, such as a valve, 210a,b,c respectively. Each flow control component 210a,b,c is associated with a respective negative pressure source 228a,b,c, which in turn controls fluid discharge into canisters or reservoirs 212a,b,c respectively. For example, the patient interface 12 can comprise an external patient interface 16 as described above and a pair of deep drainage tubes 50a,b. The patient interface 12 includes an optional supply component 214, which can comprise one or more fluid reservoirs, pumps (manual or powered) and associated controls, which can connect to the microprocessor 204 for system control. The supply component 214 optionally takes to one or more of the tubes 50, 60 for delivering fluid to the patient through the deep drainage tubes 50 or through the external patient interface 16. Such fluids can comprise, for example, antibiotics, and aesthetics, irrigating agents, growth factor, and any other fluid beneficial in promoting healing, countering infection and improving patient comfort.

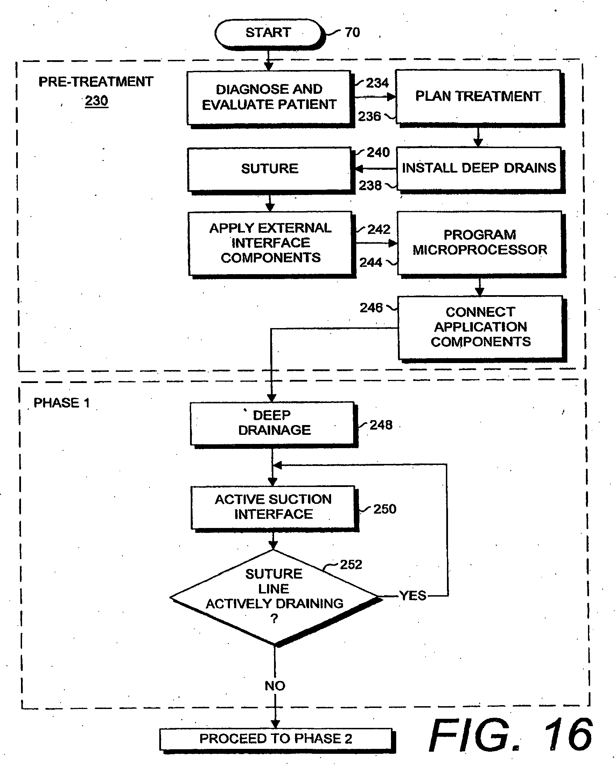

The methodology of the treatment with the alternative embodiment tissue closure system 202 is shown in Fig. 16 and generally involves modified pretreatment 230 and Phase 1 procedures. From "Start" the method proceeds to a diagnosis/evaluation step 234, a treatment plan step 236, deep drain installation 238, suturing at 240, external interface component application 242, microprocessor programming 244 and connection of the application components at 246, such as connection of the tubing. Phase 1 commences with deep drainage at 248, active suction interface at 250 and a "Suture Line Actively Draining?" decision box 252. If the suture line is actively draining, the method loops back to the active suction interface step 250, otherwise (negative determination at 252) it proceeds to Phase 2.

### V. Applications

Without limitation on the generality of useful applications of the tissue closure systems 2 and 202 of the present invention, the following partial list represents potential patient conditions and procedures, which might indicate application of the present invention.
- Over closed tissue separations, such as surgical incisions.
- Over joints where the incision is subject to movement and stretching, such as arthrotomy, reconstructive proceedures, cosmetic procedures, flaps, scar revisions, Total Joint Replacement (TJR) procedures, i.e., hip, knee, elbow, shoulder and foot.
- Any wound in an area of thick or unstable subcutaneous tissue, where splinting of skin and subcutaneous tissue might reduce dehiscence of deep sutures.
- Wounds over reconstructive procedures in which irregular cavities are created. These include resection of tumors, implants, bone, and other tissues. Changes in length and geometry of limbs, and changes in size, position, and contour of bones and other deep structures.
- Wounds in which elimination and prevention of dead space is important.
- Treatment of hematomas and seromas.
- Amputation stumps.
- Abdominal, thoracic, flank, and other wounds in which splinting of the wound might assist closing and mobilizing the patient during the postoperative interval.
- Wounds in areas of fragile or sensitive skin, where repeated removal and replacement of tape or other adhesives might produce pain, irritation, or blistering of skin in the vicinity of the wound. Also where dressing changes might produce shear or displacement of tissue so as to compromise primary wound healing.
- Wounds in cases where the patient wishes to bathe before the skin has healed sufficiently to allow protection from contamination with bath or shower water.
- Wounds subject to contamination with feces, urine, and other body fluids.
- Pediatric, geriatric, psychiatric, and neurologic patients, and other patients likely to disturb dressings and wounds.
- Patients with multiple consultants and care givers, where repeated inspection of the wound might compromise healing.
- Deep closure and surface sutures and staples.
- Any clean surgical or traumatic incision, open, or fully or partially closed by sutures, or where the skin edges can be apposed to a gap no wider than the width of the negative pressure zone of the dressing, i.e. where the maximum separation is less than or equal to the width of FTC.1 (rayon strip).
- In cosmetic and reconstructive surgery, the systems and methods of the present invention can control and conceal the effects of early bleeding, exudation, ecchymosis, and edema of the wound.
- In surgery on the limbs, where compression and drainage by this method might eliminate or reduce the need for circumferential compressive wrapping.
- Tissue separations that are prone to protracted drainage, such as hip and knee incisions, and tissue separations in patients with health conditions, such as diabetes, that tend to inhibit healing. Shortened hospital stays might result from swelling reduction and control of drainage.

### VI. Case Studies

- General concept: sequential surface application of foam material (FTC.2) to surgical site and other wounds. Air-drying at the suture line is facilitated by the rayon strip (FTC.1).
- Phase 1: deep drainage (drain tube(s)), active or passive; active suction applied to surface PUE foam (placed on top of surgical incision, drains bleeding and exudate from suture line); active suction compresses PUE foam, thus applying positive compression to the entire dissection field; adhesive-lined film underdrape with an MVTR of 3-800 on skin underlying PUE foam; rayon (or other suitable porous wicking material) strip on suture line; similar type of adhesive film overdrape (MVTR of 3-800) overlying PUE foam material.
- Duration: approximately 2-3 days, i.e. effective time for active drainage from incision/stitch line to cease and for suture line to dry and heal.
- Phase 2: Remove active suction by cutting off (elbow) connector and leave FTC.2 in place.

Released from suction, FTC.2 expands against the overdrape and exerts positive pressure differential on the operation site. May maintain continued mild compression throughout Phase 2; residual drainage function through rayon strip and into FTC.2 provides continued drying of suture line. Deep drain tubes remain in place during Phase 2 for active deep drainage.
- Duration: approximately three days, i.e. days 3-6 after operation.
- Phase 3: remove overdrape and FTC.2; leave underdrape and rayon strip in place; visually observe wound healing progress; transparency desirable.
- Duration: several (e.g., up to three) weeks.
- Clinical trial confirmation: Closure of surgical site in upper chest area in patient with severe healing problems showed excellent results and rapid wound healing.
- Subcuticular (subepidermal) sutures avoid conflict with rayon strip and need for early suture removal, or pressure on skin sutures beneath compressive black sponge.
- Option: use pressure transducer for interface pressure mapping of wound site and automate control and monitor pressures, flow, etc.

### VII. Alternative Embodiment Tissue Closure System 302.

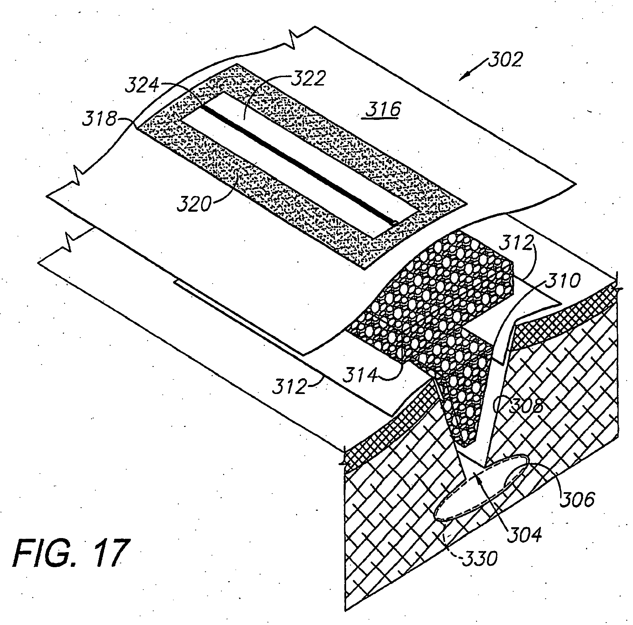

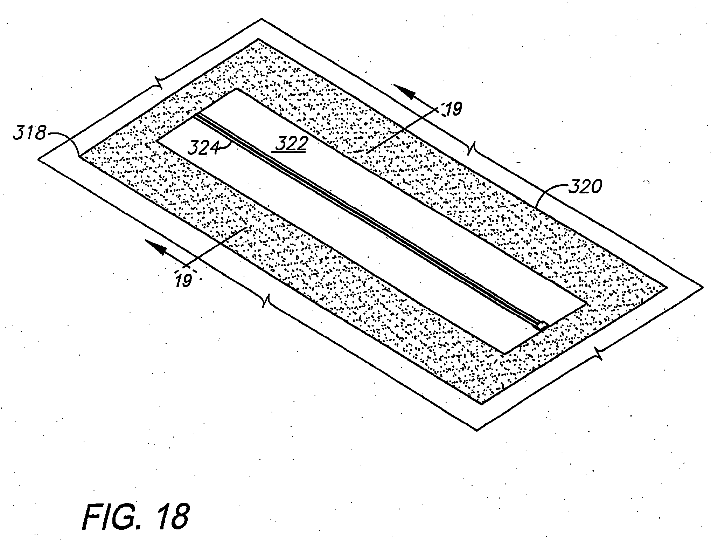

A tissue closure system 302 comprising an alternative embodiment of the present invention is shown in Figs. 17-22. The system 302 is adapted for closing a wound 304 with an undermined area 306 just above the fascia and an upper tissue separation 308 located primarily in the dermis and in the subcutaneous layer. A wedge-shaped internal fluid transfer component (foam piece) 310 is located in the tissue separation area 308 and is installed between side drapes 312 located on either side of the wound 304. An external fluid transfer component (foam piece) 314 is placed on top of the internal component 310 and the side drapes 312, and is covered by an outer drape 316. An optional innermost foam piece 330 can be located in and sized to fit the undermined area 306 and can transfer fluid and gradient forces to and from the internal foam piece 310.

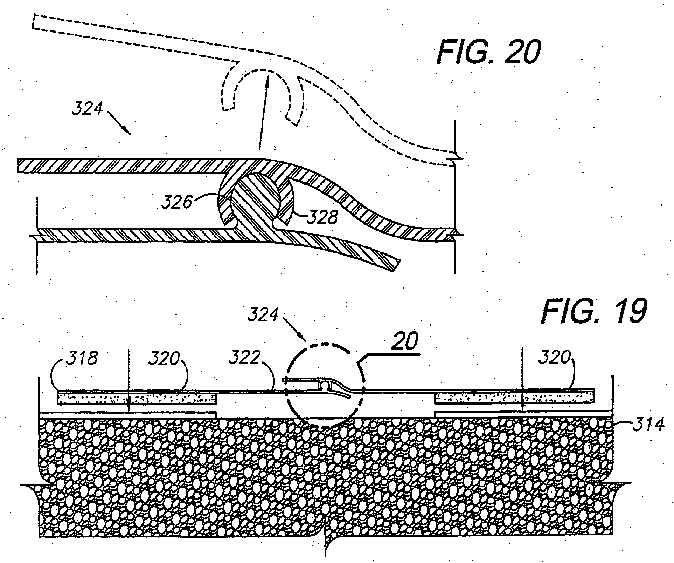

A reclosable access panel 318 is placed over an opening formed in the outer drape 316 and includes an adhesive-coated perimeter 320 surrounding an adhesive-free center area 322 with a reclosable seal strip 324 extending longitudinally down the centerline thereof. The seal strip 324 includes a rib or bead 326, which is releasably captured in a channel 328 (Fig. 20).

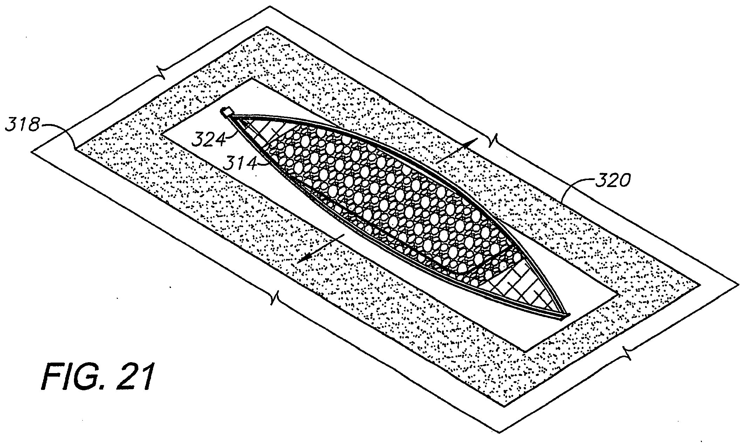

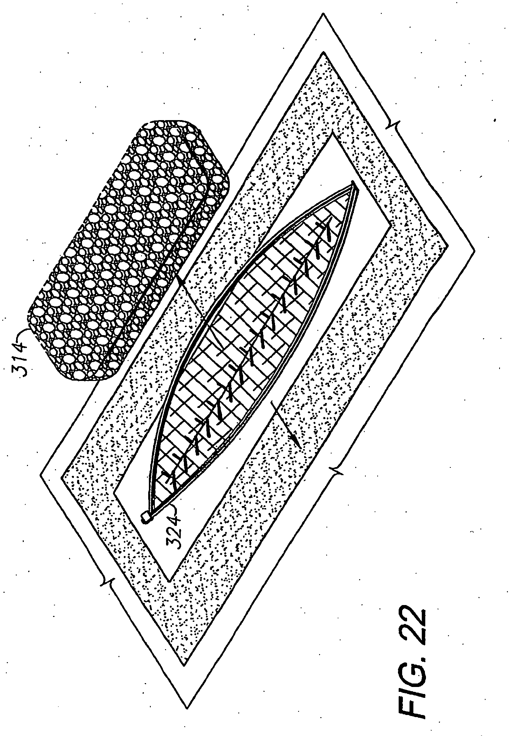

In operation, the reclosable access panel 318 is adhesively secured around its perimeter 322 to the outer drape 316 and provides access to the foam pieces 310, 314 of the dressing system 302. For example, the foam pieces 310, 314 can be changed (Figs. 21 and 22), treatments can be applied and wound healing progress can be visually monitored.

### VIII. Alternative External Dressing 402.

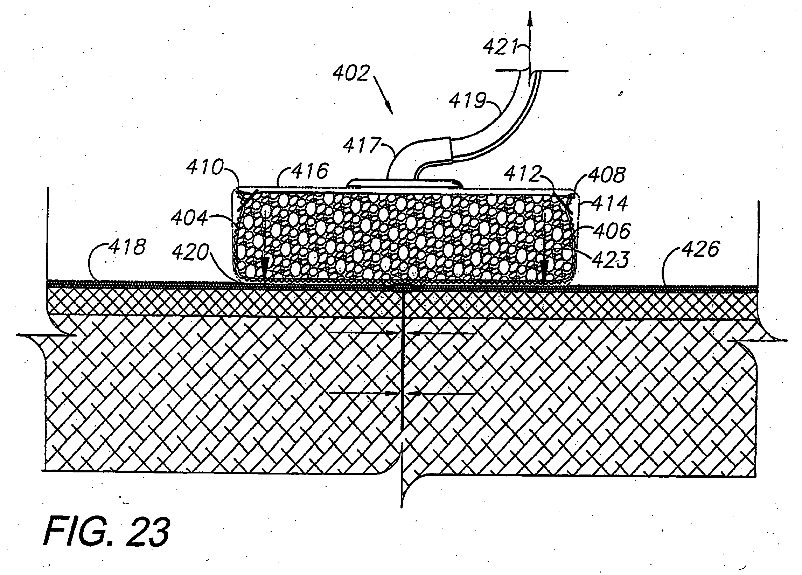

Figs. 23-27 show an external dressing 402, which can be premanufactured or preassembled and used for various wound treatment and closure applications. The dressing 402 includes a foam piece 404 partially enclosed in a rayon covering 406, which includes an open top 408 secured to an upper perimeter 410 of the foam piece 404, for example, by sutures, staples, adhesive or some other suitable mechanical fastener as shown at 412. The dressing 402 is preferably preassembled with an outer drape 414 including a foam-covering central portion 416 and a perimeter, patient-contact skirt portion 418. A tucked margin 420 is formed at the intersection of the drape portions 416, 418 and partially underlies the foam piece 404 in order to protect the skin and prevent the formation of low-pressure, vacuum voids around the edge of the foam piece 404 whereat blistering could otherwise occur. In operation, the dressing 402 can be easily changed by cutting around the margin 420, removing the foam piece 404 and the drape outer portion 416. The wound can thus be inspected, cleaned, debrided, treated, etc. and a new dressing 402 put in place. The patient-contact skirt portion 418 of the original dressing can remain in place.

Fig. 23 shows a fluid flow (discharge) directional arrow 421 from an elbow coupling 417 and a discharge tube 419. Alternatively, fluid could be injected into the dressing 402 through the tube 419 and the coupling 417. Hydraulic/pneumatic compressive force arrows 423 are shown in Fig. 23 and represent the downward (i.e. into patient) forces, which can be established by compressing the foam piece 404 under suction and then releasing the negative pressure differential, thus transitioning the dressing to a positive pressure differential. In a positive pressure differential mode of operation, the dressing 402 controls edema by pressing the foam piece 404 against the tissue adjacent to the wound. There are many potential medical benefits from controlling edema in this manner. For example, healing is promoted, scar tissue is minimized and patient discomfort can be reduced.

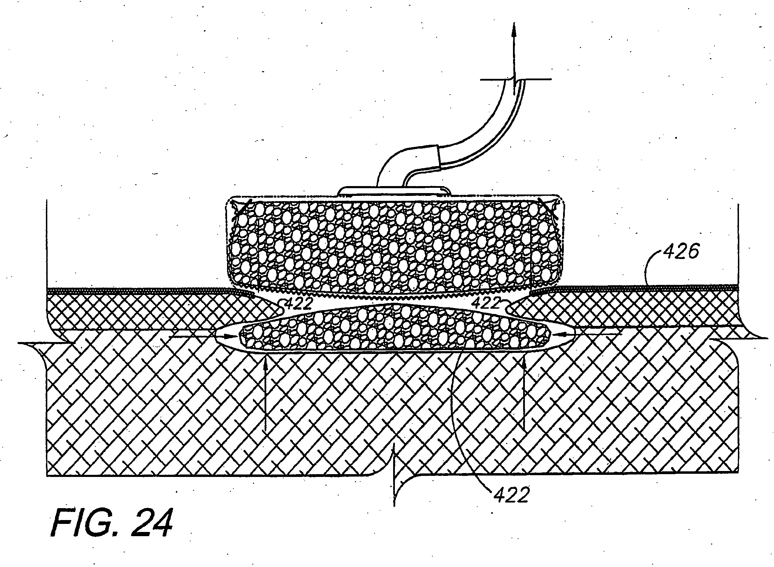

Fig. 24 shows the external dressing 402 used in conjunction with an internal foam piece 422, which is located below the dermis at the top of the subcutaneous layer. The internal foam piece 422 is adapted for applying a pressure differential within the subcutaneous layer whereby tissue growth and closure are promoted. The inside/outside configuration of the dressing system shown in Fig. 24 can rehabilitate and make pliable a wound edge 424 that has contracted and become hard, immobile and edematous by applying pressure differentials across the external and internal foam pieces 404, 422, such as compression (positive pressure differential) for edema control.

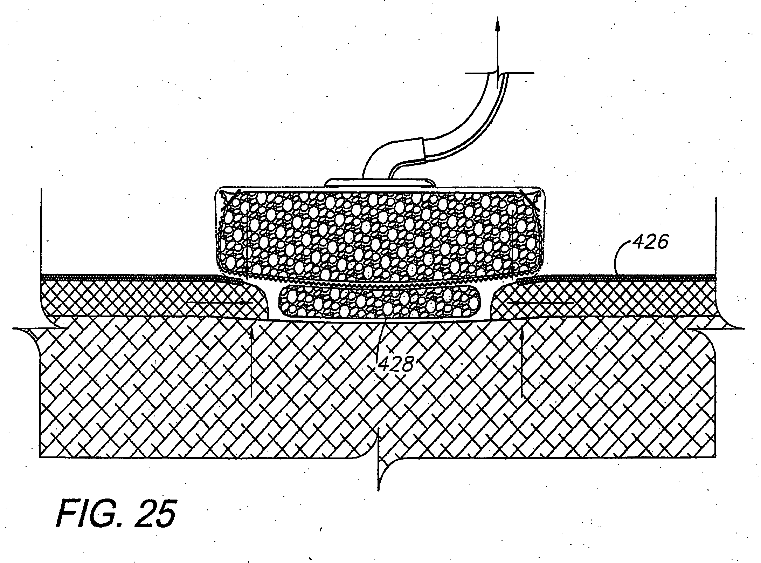

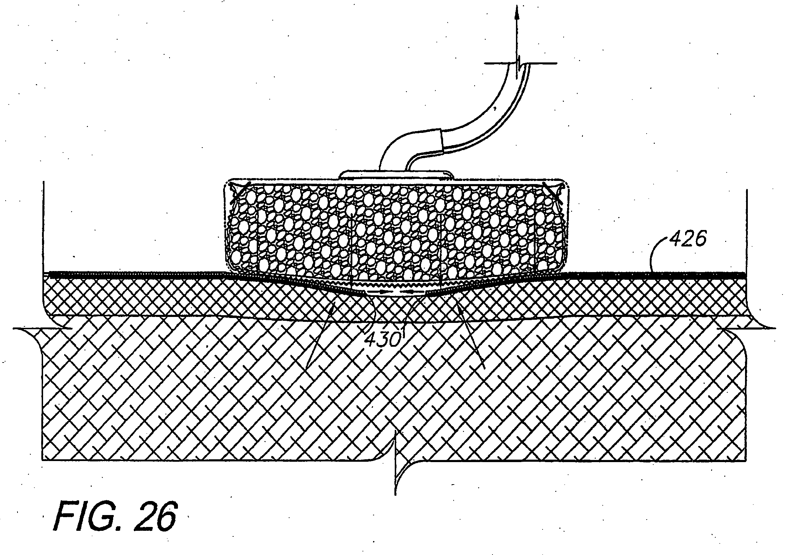

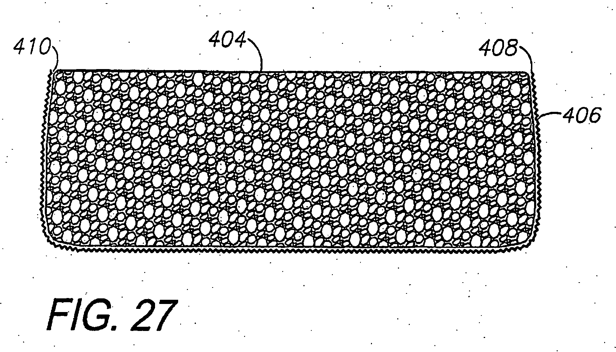

Fig. 25 shows the wound confined to the dermis 426 with another internal foam piece 428 in place. The subcutaneous layer is substantially healed. Fig. 26 shows the external foam piece 404 in place alone for drawing the wound edges 430 together at the epidermis. Fig. 27 shows the external foam piece 404 covered on the sides and bottom by the rayon covering 406, leaving an open top 408.

### IX. Alternative Embodiment Dressing System 502

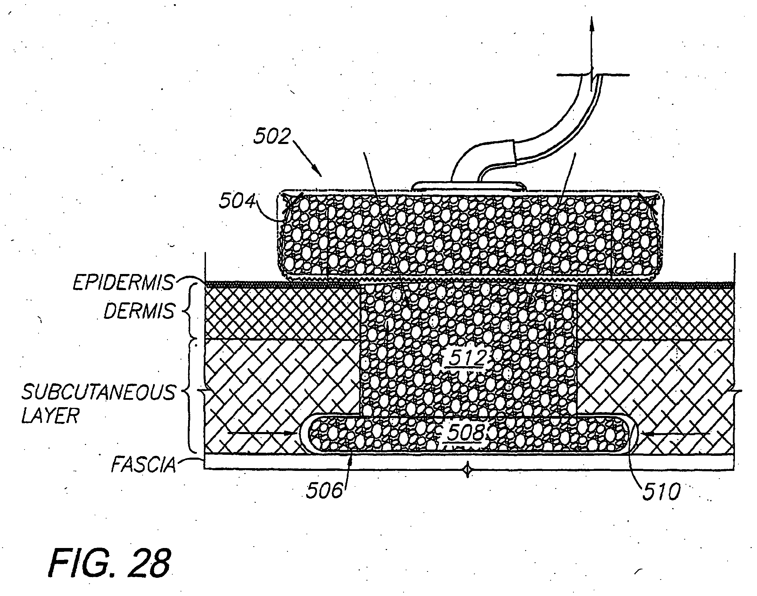

Fig. 28 shows yet another alternative embodiment internal/external dressing system configuration 502 with an external foam piece 504 similar to the foam piece 404 described above and an internal foam assembly 506 located in the dermis and in the subcutaneous layer. The assembly 506 consists of a proximate internal foam piece 508, which can be located at the bottom of the subcutaneous layer on top of the fascia in an undermined cavity 510 formed by the wound, and a distal internal foam piece 412 located primarily in the dermis and the subcutaneous layer portions of the wound between the external foam piece 504 and the proximate internal foam piece 508.

The dressing system configuration 502 can be configured and reconfigured as necessary to accommodate various wound configurations in various stages of healing. For example, the proximate internal foam piece 508 can be removed when the undermined cavity 510 closes. Likewise, the distal internal foam piece 512 can be removed when the subcutaneous layer and the dermis have healed. Moreover, the foam pieces 504, 508 and 512 can be replaced with different sizes of foam pieces as necessary in connection with dressing changes and as the wound configuration changes. Such sizes and configurations can be chosen to optimize the beneficial effects of pressure gradients (both positive and negative), fluid control, edema control, antibacterial measures, irrigation and other treatment protocols. Still further, the access panel 318 described above can be used in conjunction with the dressing system 502 in order to provide access to the foam pieces thereof and to the wound itself.

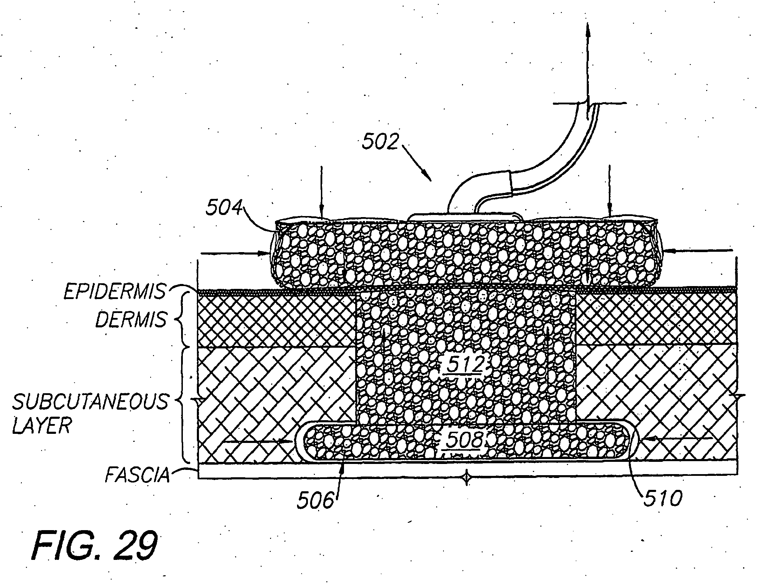

Fig. 29 shows the internal/external dressing system 502 compressed under the vacuum effects of an external vacuum source with the drape 316 drawn tightly down on the compressed outer foam piece 504. Thus compressed, the system 502 is adapted to transfer positive pressure differential, compressive forces to the area of the wound.

### X. Alternative Embodiment Dressing Assembly 602

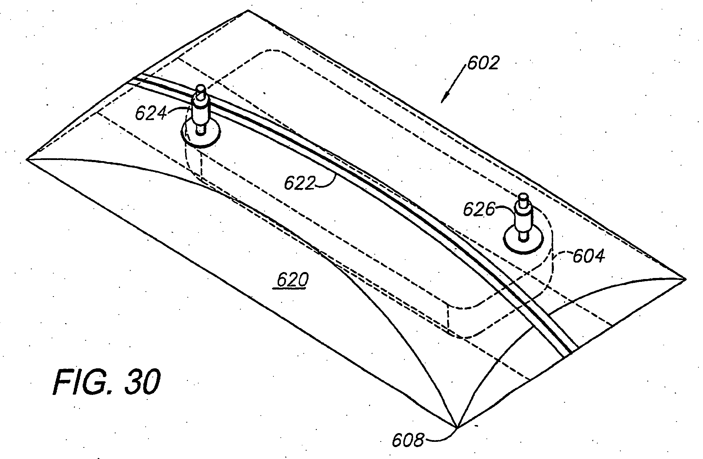

Figs. 30-37 show a reclosable, preassembled external dressing assembly 602 comprising an alternative embodiment of the present invention. The dressing assembly 602 includes a foam piece 604, which can be completely covered in rayon 606 or some other suitable material with the desired absorbent and/or wicking capabilities. The foam piece 604 also includes a core 605 comprising a suitable material, such as polyurethane, hydrophobic foam. Alternatively, other foam materials with hydrophobic or hydrophilic properties can be utilized. Various sizes and shapes of the foam piece 604 can also be employed, including cutting and trimming it to size during the course of a medical procedure.

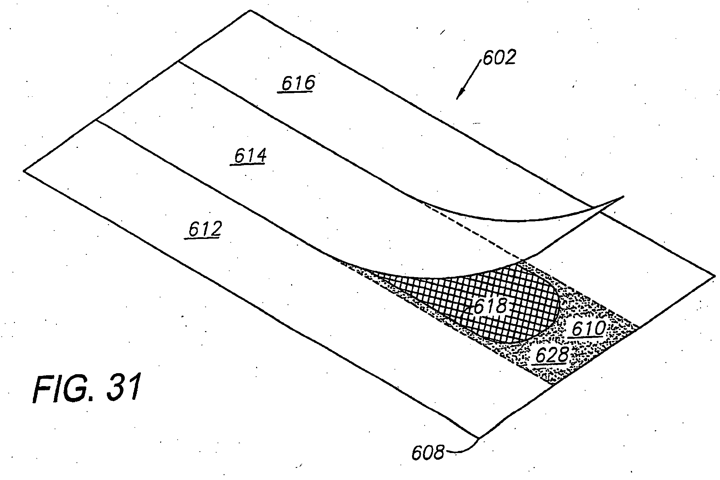

The foam piece 604 is removably placed in a reclosable sheath 608 including a bottom panel 610 selectively covered by removable, adhesive backing strips 612, 614 and 616 forming a central opening 618. As shown in Fig. 31, a central opening 618 in the bottom panel 610 is initially covered by the center backing strip 614. Removing the center backing strip 614 exposes the foam piece 604 through the opening 618. The reclosable sheath 608 also includes a top panel 620 with a reclosable seal strip 622 extending from end-to-end and generally longitudinally centered. The seal strip 622 can be similar in construction to the reclosable seal strip 324 described above. The top panel 620 also includes fluid ports 324, 326, which can comprise, for example, Leur lock connectors or some other suitable fluid connection device.

The sheath 608 can comprise polyethylene or some other suitable material chosen on the basis of performance criteria such as permeability, flexibility, biocompatibility and antibacterial properties. Various permeable and semi-permeable materials are commonly used as skin drapes in medical applications where healing can be promoted by exposure to air circulation. The sheath 608 can be formed from such materials for applications where continuous vacuum suction is available and the dressing 602 is not required to be airtight.

According to an embodiment of the method of the present invention, a dressing assembly 602 can be premanufactured, or custom-assembled from suitable components for particular applications. In a premanufactured version, the dressing 602 is preferably presterilized and packaged in sterile packaging.

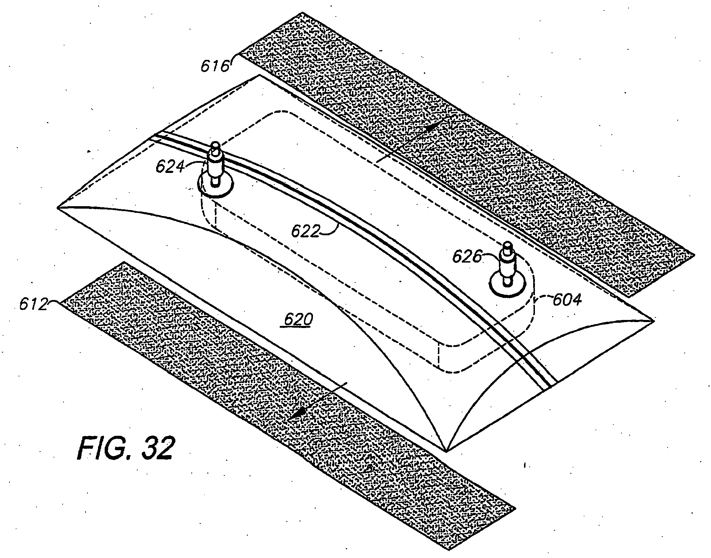

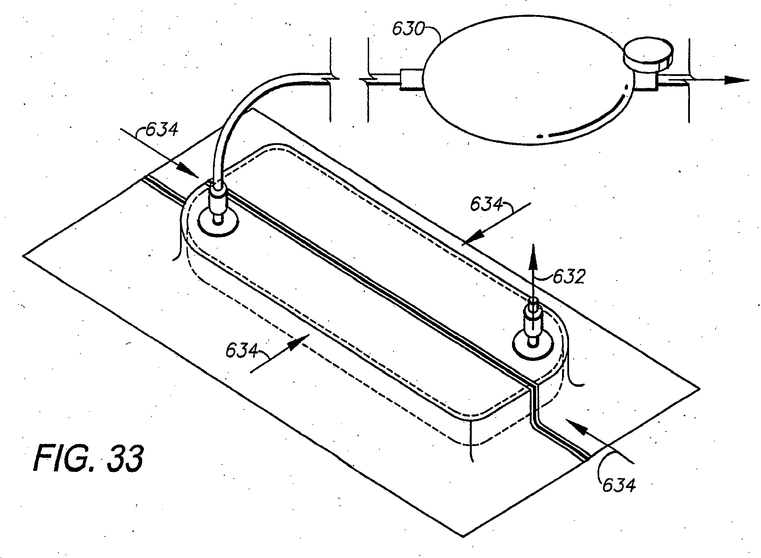

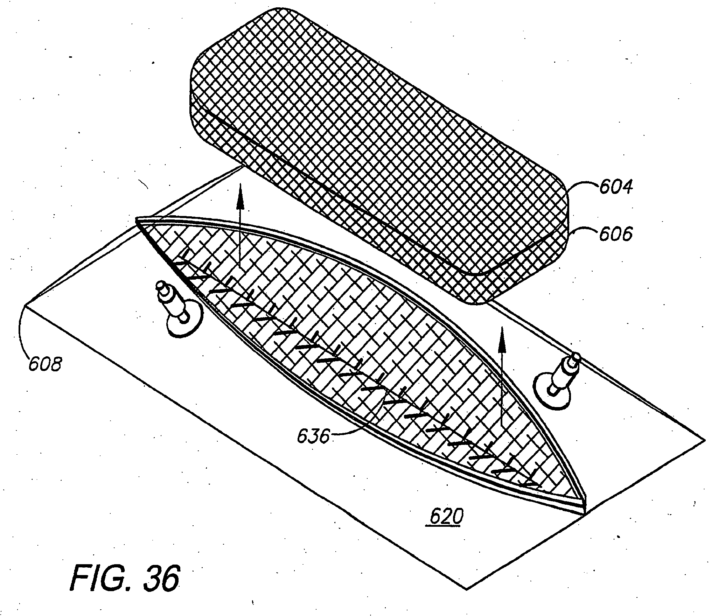

A common application of the dressing 602 is on a recently-closed surgical incision for controlling bleeding and other fluid exudate. For example, the dressing 602 can be placed on the patient with its bottom panel opening 618 located over a stitch line 636 (Fig. 36). The center backing strip 614 is peeled from the bottom panel 610 to expose the opening 618 and the adhesive 628 on the bottom panel 610 (Fig. 33). The opening 618 provides a fluid transfer, which can also be provided by constructing the sheath bottom panel 610 from a permeable material, or by providing other passage configurations therethrough. The dressing 602 can then be placed on the patient, with the bottom panel adhesive providing temporary fixation. The side backing strips 612, 616 can then be removed, as shown in Fig. 32, and the bottom panel 610 completely secured to the patient.

The fluid ports 624, 626 are adapted for either extraction or infusion of fluids, or both, depending on the particular treatment methodology. For extraction purposes a vacuum source can be attached to one or both of the ports 624, 626, and can comprise a mechanical, powered pressure differential source, such as wall suction. Alternatively, hand-operated mechanical suction can be provided, such as a suction bulb 630 (Fig. 33) or a Hemovac device available from Zimmer Corp. of Warsaw, Indiana. Such hand-operated suction devices can accommodate patient mobility and tend to be relatively simple to operate. Powered suction and fluid pump devices can be preprogrammed to provide intermittent and alternating suction and infusion, and to automatically respond to patient condition feedback signals. As shown in Fig. 33, the application of a negative pressure differential (suction) collapses the sheath 608 onto the foam piece 604. The various dynamic fluid forces and fluid movement effects described above can thus be brought into operation and controlled.

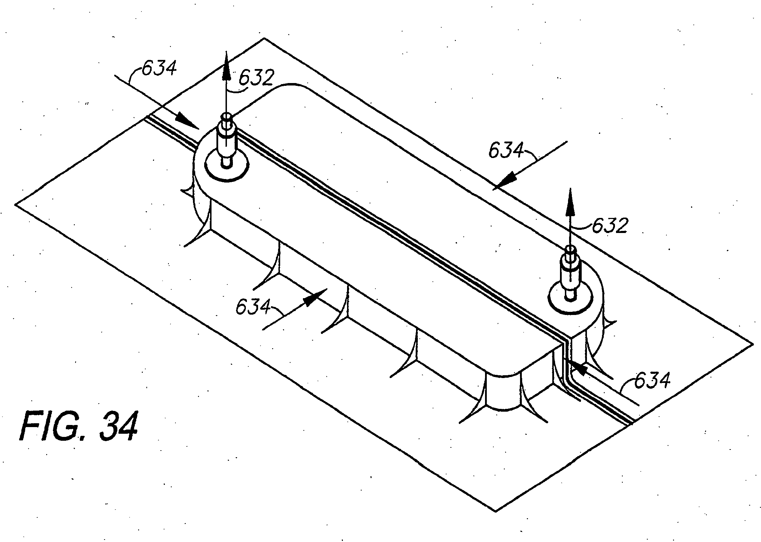

Fig. 34 shows the sheath 608 further collapsing on the foam piece 604 as a result of evacuation from both of the fluid ports 24, as indicated by the fluid flow arrows 632. The ambient air pressure force arrows 634 show the application of this force, which tends to collapse the sheath 608 onto the foam piece 604.

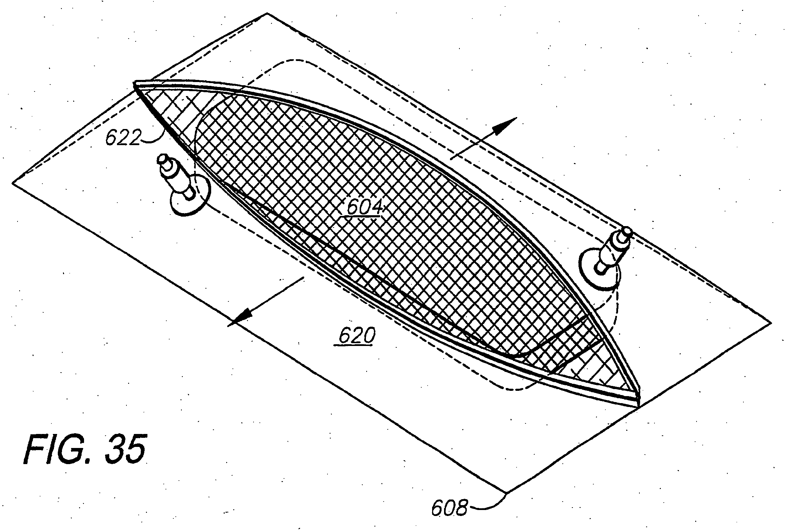

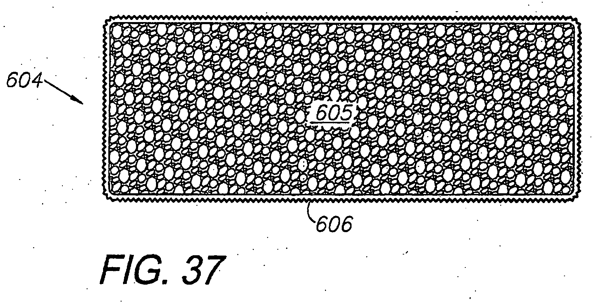

Fig. 35 shows opening the seal strip 622 for access to the interior of the dressing 602. The foam piece 604 can then be removed, as shown in Fig. 36, whereby the stitch line 636 can be visually inspected and/or treated. The foam piece 604 can be flipped over or replaced, as necessary. Fig. 37 shows a cross-section of the foam piece 604, which can be completely covered in rayon or some other suitable wicking material 606 in order to accommodate placement of either side against the stitch line 636.

### XI. Alternative Embodiment Dressing Assembly 702

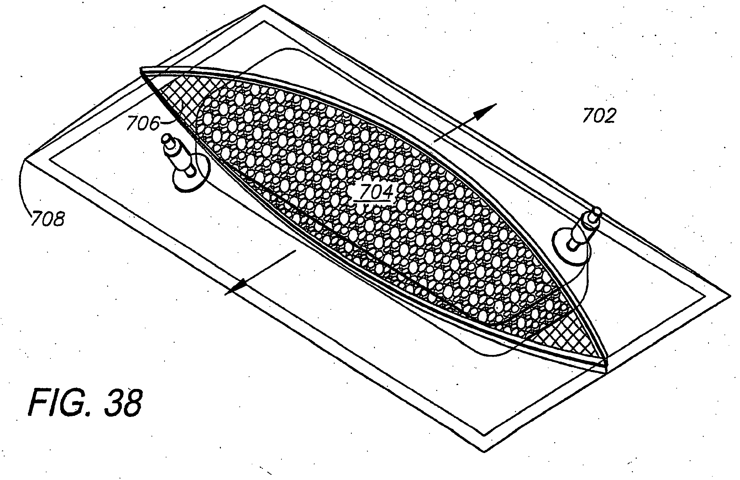

Figs. 38-40 show a dressing assembly 702 comprising an alternative embodiment of the present invention and including a foam piece 704 comprising any suitable hydrophobic or hydrophilic foam material. The foam piece 704 is selectively and removably located in a sheath 708, which can be similar to the sheath 608 described above. A liner 706 can comprise a piece of rayon or some other suitable material adapted to wick fluid from the stitch line 636 into the foam piece 704, and further adapted to isolate the patient from direct contact with the foam piece 704. The liner 706 can be sized to lay flat against the bottom panel of the sheath 708.

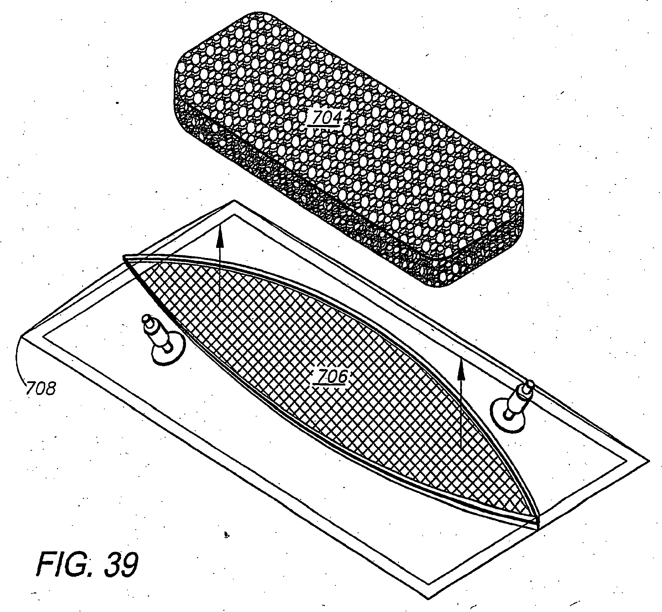

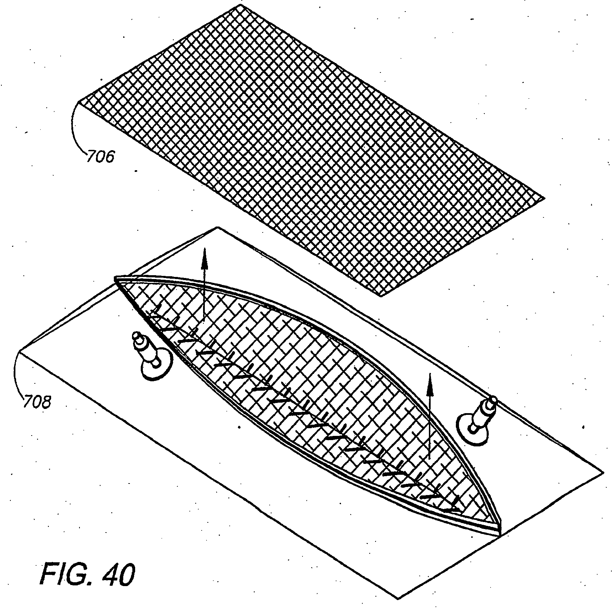

In operation, the dressing assembly 702 is adapted to utilize readily available components, such as the foam piece 704 and the liner 706, in a dressing adapted for wound inspection, wound treatment and component change procedures, all without having to remove the sheath or disturb its adhesive attachment to the patient. Fig. 39 shows removing the foam piece 704, which can be flipped over for reuse or replaced. Fig. 40 shows removing the liner 706, which can also be easily replaced. With the liner 706 removed, the stitch line 636 is exposed for stitch removal, inspection, treatment, irrigation and other procedures. The sheath 708 can then be reclosed and vacuum-assisted and/or other treatment can resume.

### XII. Alternative Embodiment Dressing Assembly 802

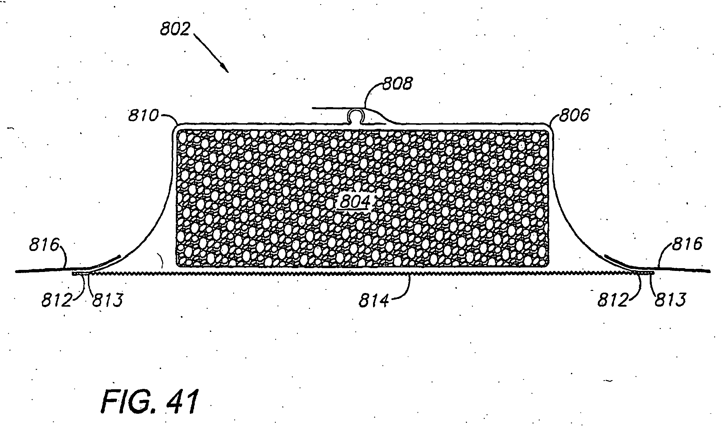

A dressing assembly 802 comprising an alternative embodiment of the present invention is shown in Fig. 41 and includes a foam piece 804 in a sheath 806 adapted for opening and closing through a reclosable seal strip 808. The sheath 806 includes an upper drape portion 810, which can comprise a suitable semi-permeable or impervious drape material. The sheath 806 includes a perimeter 812, which can be provided with an optional adhesive perimeter seal 813 adapted for providing a relatively fluid-tight seal around the sheath 806. The perimeter seal 813 can be relatively narrow in order to minimize patient discomfort, skin maceration, etc. A bottom panel 814 comprises a suitable wicking material, such as rayon, and extends to the sheath perimeter 812. The materials comprising the dressing 802 can be chosen for permeability or occlusiveness, biocompatibility, hydrophobic or hydrophilic reaction to liquids, bacteriastatic and antimicrobial properties, and other performance-related properties and criteria.

In operation, the dressing 802 is placed on the patient over a wound or stitch line. The perimeter adhesive 813 can provide temporary fixation and sealing. A strip of tape 816 can be placed over the sheath perimeter 812 for securing the sheath 806 in place. Fluid is transferred through the wicking material layer 814 to the foam piece 804 for evacuation through suitable fluid connectors, as described above, which can be attached to a vacuum source. Moreover, the dressing 802 is adapted for providing a positive pressure gradient, also as described above. The seal strip 808 permits access to the foam piece 804 for flipping over or changing, as indicated.

The foam piece 804, the drape upper portion 810 and the wicking material layer 814 can be assembled for independent movement whereby the only attachment among these components occurs around the perimeter 812 where the drape upper portion 810 is connected to the wicking material layer 814. Such independent freedom of movement permits the dressing assembly 802 to reconfigure itself and conform to the patient and various applied forces, such as pressure gradients. The individual components can thus expand and contract independently of each other without distorting the other components or interfering with the performance and comfort of the dressing assembly 802.

### XIII. Alternative Embodiment Dressing System 902

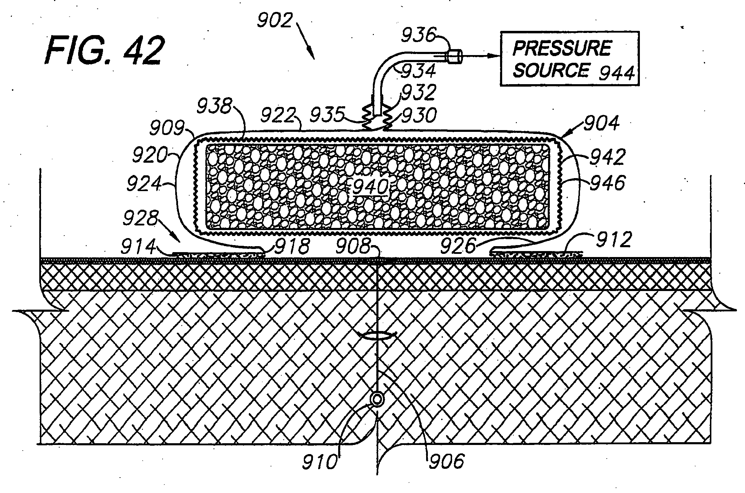

A dressing system 902 comprising another alternative aspect or embodiment of the present invention is shown in Figs. 42-46 and includes a dressing 904 adapted for controlling the application of positive, compressive forces and/or negative, suction forces to a patient with an incision-type tissue separation 906. Without limitation of the generality of useful applications of the system 902, the incision 906 can comprise a surgical incision, which can optionally be closed with stitches 908 or other suitable wound-closure procedures, including staples, adhesives, tapes, etc. The incision 906 can include a closed suction drainage tube 910 in the base of the incision, which can be brought to the skin surface through a stab incision, using well-known surgical procedures.

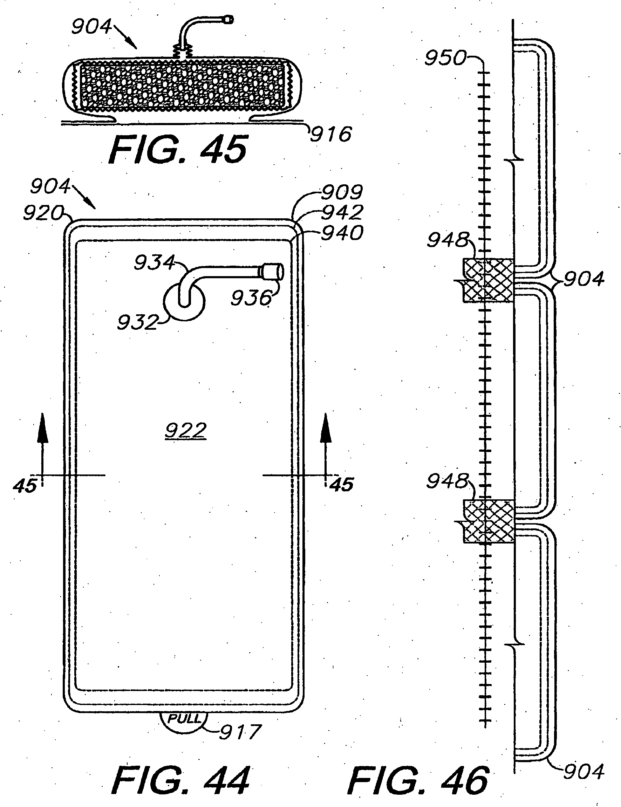

The dressing 904 includes a dressing cover 909 with an optional perimeter base ring 912, which comprises a semi-permeable material with a layer of skin-compatible adhesive 914 applied to a lower face thereof. Prior to application of the dressing 904, the base ring adhesive 914 mounts a release paper backing 916 (Fig. 45) with a release tab 917 (Fig. 44). The base ring 912 defines a central, proximal opening 918, through which the dressing 904 is downwardly open. A cover superstructure 920 includes a distal panel 922, a perimeter 924 generally defining a folding, collapsible edge, and a proximal return ring 926 secured to the base ring 912 around the central opening 918 at another folding, collapsible edge. The base and return rings 912, 926 thus form an invaginated, double-thickness base structure 928 adapted to expand and collapse. A distal cover opening 930 is formed in the distal panel 922 and communicates with a flexible, bellows-shaped collapsible sheath, which in turn mounts a length of rigid tubing 934 terminating distally in a connector 936 comprising, for example, a needle-free, leur lock hub or other suitable tubing connection/closure device, such as an air valve. The tubing 934 includes a proximal end 935 communicating with the interior of the dressing cover 909

An optional transfer assembly or element 938 is positioned within the cover 909 and is exposed through the central opening 918 thereof. The transfer assembly 938 optionally includes a compressible, reticulated core 940, which can comprise, for example, polyurethane ether foam material chosen for its hydrophobic, resilient and memory performance characteristics. The transfer assembly 938 also includes a porous, flexible liner 942 comprising a material such as Owens^{®} rayon surgical dressing with liquid-wicking properties and biocompatibility for direct contact with patients' skin.

Without limitation on the generality of useful applications of the dressing system 902, post-operative incision dressing applications are particularly well-suited for same. The dressing 904 can be preassembled and sterile-packaged for opening under sterile conditions, such as those typically maintained in operating rooms. The central opening 918 can be sized to accommodate the tissue separation 906 with sufficient overlap whereby the perimeter base ring adhesive 914 adheres to healthy skin around the area of the tissue separation 906 and beyond the area of underlying internal operative dissection. Multiple dressings 904 can be placed end-to-end (Fig. 46) or side-by-side in order to effectively cover relatively long incisions 950. In such multiple dressing applications, the stitch line 952 can be covered with an intervening barrier layer strip 948 at locations where the adhesive-coated base ring crosses same for purposes of patient comfort. The barrier layer strips 948 can comprise, for example: Xeroform^{®} gauze available from Integrity Medical Devices, Inc. of Elwood, New Jersey; Vaseline^{®} gauze; or straps of Owens^{®} rayon,.

The base ring adhesive 914 preferably forms a relatively fluid-tight engagement around the treatment area. Optionally, the base ring 912 can comprise a suitable semi-permeable membrane material, with suitable breathability characteristics for enhancing patient comfort and avoiding maceration in the contact areas. A suitable differential pressure source 944 is coupled to the tubing connector 936. Without limitation, the pressure source 944 can comprise automated and manual pressure sources. For example, automated wall suction is commonly available in operating rooms and elsewhere in health-care facilities.

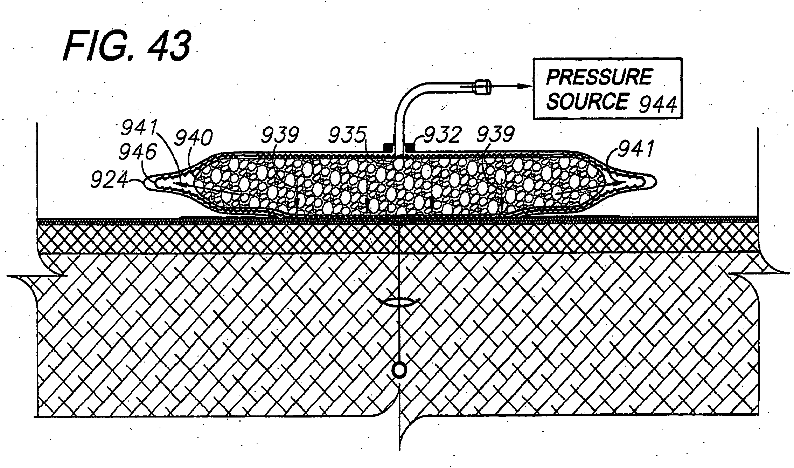

For post-operative incision dressings, operating room wall suction can be attached to the connector 936, the dressing 904 evacuated, and the wall suction disconnected whereby the connector 936 seals the system. It will be appreciated that a "steady-state" condition of equilibrium can be achieved with positive, ambient air pressure acting externally on the dressing cover 909 and the transfer assembly 938 compressed internally, and thus exerting compressive forces on the incision 906 and the surrounding area via compressive force arrows 939 (Fig. 43).

For example, Fig. 43 shows the dressing 904 collapsed with the rayon dressing liner 942 extending beyond the polyurethane ether foam core 940 and forming a double-thickness liner perimeter 946 located within the double-folded cover perimeter 924. In this configuration any liquid exudate from the incision 906 is effectively transferred by wicking action of the rayon liner 942 away from the incision 906 via fluid transfer arrows 941. Serosanguineous fluid emissions can be expected from an incision line for a short period, commonly a day or two, after an operation. The wicking action of the rayon liner 942, coupled with the slight ambient air circulation admitted through the semi-permeable base ring 912, cooperate to maintain the incision 906 and the healthy skin around it relatively dry in order to avoid maceration. The pressure differential provided by components of the dressing 904 can also contribute to extraction and removal of wound exudates, in cooperation with the wicking action described above. With the dressing 904 in its compressed configuration (Fig. 43), the tubing proximal end 935 can engage and be pushed into the transfer element 938 for direct fluid transfer therebetween.

The evacuated dressing 904 provides a number of medical incision-closure and healing benefits. The stabilizing and fixating effects on the incision and the surrounding tissue resulting from the forces applied by the dressing 904 tend to promote contact healing, as opposed to gap healing or healing wherein opposing edges are sliding and moving one on the other. Moreover, edema and ecchymosis control are accomplished by exerting positive pressure, compressive force via the compressive force arrows 939 in the compressed core 940, which tends to resume its precompression shape and volume as pressure is released within the dressing 904. Thus, the effects of restricted or controlled leakage, for example around the base ring 912, tend to be offset by the controlled expansion of the core 940. The limited air movement through the dressing 904 can be beneficial for controlling internal moisture, reducing maceration, etc.

The system 902 is adapted for adjustment and replacement as necessary in the course of closing and healing an incision. Additional air displacement can be applied via the connector 936 from automated or manual sources. Wall suction, mechanized pumps and other automated sources can be applied. Manual vacuum sources include: squeeze-type bulbs (630 in Fig. 33); (Snyder) Hemovac^{®} evacuators available from Zimmer, Inc. of Warsaw, Indiana; and vacuum tubes. Inspection of the incision 906 can be accomplished by making an L-shaped cut in the dressing cover superstructure 920 and extracting or lifting the transfer assembly 938, thereby exposing the incision 906. The transfer assembly 938 can be flipped over or replaced. The dressing 904 can then be resealed by applying a replacement portion of the cover 909, whereafter the dressing 904 can be evacuated as described above. After treatment is completed, the cover superstructure 920 can be cut away and the transfer assembly 938 can be discarded. The base ring 912 can be peeled away from the skin, or simply left in place until the adhesive 914 releases.

The stabilizing, fixating and closing forces associated with the dressing 904 tend to facilitate healing by maintaining separated tissue portions in contact with each other, and by controlling and/or eliminating lateral movement of the tissue, which can prevent healing. The positive pressure, compressive force components associated with the forces in the dressing 902 tend to close the tissue separation 906 and retain the opposing tissue edges in fixed contact with each other whereby healing is promoted. Various other dynamic forces tending to displace the wound edges relative to each other can be effectively resisted.

It is to be understood that while certain embodiments and/or aspects of the invention have been shown and described, the invention is not limited thereto and encompasses various other embodiments/aspects as defined by the claims.

## Claims

1. A dressing assembly (302) for a closed wound or incision (304), which comprises:
an external patient interface including an external fluid transfer component (314), said external fluid transfer component (314) being adapted for transferring fluid from the closed wound or incision (304);
said external patient interface including an over-drape (316) placed over said external fluid transfer component (314) in contact with a surrounding skin surface;
a re-closeable seal strip (324) connected to said over-drape (316) and having a closed position sealing same and an open position providing access to said external fluid transfer component (314) therethrough;
a pair of side drapes (312) each located along a respective side of said wound or incision (304) between said external fluid transfer component (314) and the patient's skin;
a pressure source (28) connected to the external fluid transfer component (314); and
an internal fluid transfer component (310) for location in said wound or incision in fluidic communication with said external fluid transfer component (314).

2. The dressing (302) according to Claim 1 wherein said over-drape (316) having an opening located over said fluid transfer component (314); there being an access panel (318) including said seal strip (324) and mounted on said over-drape (316) with said seal strip (324) selectively providing access to said fluid transfer component (314) through said over-drape (316) opening; said access panel (318) having a perimeter portion (320) with an adhesive coating adapted for attaching same to said over-drape (316); and said access panel (318) having an adhesive-free central portion (322) surrounded by said perimeter portion (320), said central portion (322) including said seal strip (324).

3. The dressing assembly (302) according to any one of Claims 1 or 2 wherein said recloseable seal strip (324) includes a longitudinally-extending channel (328) mounted on a first portion of said access panel (318) and a longitudinally-extending rib (326) mounted on a second portion of said access panel (3 18) and selectively received in said channel (328) with said seal strip (324) in its closed configuration.

## Patentansprüche

1. Wundabdeckungsanordnung (302) für eine geschlossene Wunde oder einen geschlossenen Schnitt (304), Folgendes umfassend:
eine externe patientenseitige Auflage, die eine externe Flüssigkeitsableitkomponente (314) aufweist, wobei die externe Flüssigkeitsableitkomponente (314) zum Ableiten von Flüssigkeit aus der geschlossenen Wunde oder dem geschlossenen Schnitt (304) eingerichtet ist;
wobei die externe patientenseitige Auflage eine obere Abdeckung (316) aufweist, die über der externen Flüssigkeitsableitkomponente (314) in Kontakt mit einer umgebenden Hautoberfläche angeordnet ist;
einen wiederverschließbaren Dichtungsstreifen (324), der mit der oberen Abdeckung (316) verbunden ist und eine geschlossene Position, die die Abdeckung abdichtet, und eine offene Position aufweist, die durch die Abdeckung hindurch Zugang zu der Flüssigkeitsableitkomponente (314) bereitstellt;
ein Paar seitlicher Abdeckungen (312), die jeweils entlang einer Seite der Wunde oder des Schnitts (304) zwischen der externen Flüssigkeitsableitkomponente (314) und der Haut des Patienten angeordnet sind;
eine Druckquelle (28), die mit der externen Flüssigkeitsableitkomponente (314) verbunden ist; und
eine interne Flüssigkeitsableitkomponente (310) zur Anordnung in der Wunde oder dem Schnitt in Flüssigkeitsverbindung mit der externen Flüssigkeitsableitkomponente (314).

2. Wundabdeckung (302) nach Anspruch 1, wobei die obere Abdeckung (316) eine Öffnung aufweist, die über der Flüssigkeitsableitkomponente (314) angeordnet ist; wobei eine Zugangsblende (318) vorhanden ist, die den Dichtungsstreifen (324) aufweist und derart an der oberen Abdeckung (316) befestigt ist, dass der Dichtungsstreifen (324) selektiv durch die Öffnung der oberen Abdeckung (316) hindurch Zugang zu der Flüssigkeitsableitkomponente (314) bereitstellt; wobei die Zugangsblende (318) einen Umfangsabschnitt (320) mit einer Klebstoffbeschichtung aufweist, der zum Befestigen desselben an der oberen Abdeckung (316) eingerichtet ist; und wobei die Zugangsblende (318) einen klebstofffreien mittleren Abschnitt (322) aufweist, der von dem Umfangsabschnitt (320) umgeben ist, wobei der mittlere Abschnitt (322) den Dichtungsstreifen (324) aufweist.

3. Wundabdeckungsanordnung (302) nach einem der Ansprüche 1 oder 2, wobei der wiederverschließbare Dichtungsstreifen (324) einen längs verlaufenden Kanal (328), der an einem ersten Abschnitt der Zugangsblende (318) befestigt ist, und eine längs verlaufende Rippe (326) aufweist, die an einem zweiten Abschnitt der Zugangsblende (318) befestigt ist und selektiv in dem Kanal (328) aufgenommen ist, wenn sich der Dichtungsstreifen (324) in seiner geschlossenen Position befindet.

## Revendications

1. Ensemble formant pansement (302) pour une plaie fermée ou une incision (304), qui comprend :
une interface patient extérieure incluant un composant de transfert de fluide externe (314), ledit composant de transfert de fluide externe (314) étant adapté pour transférer le fluide de la plaie fermée ou l'incision (304) ;
ladite interface patient extérieure incluant un champ de recouvrement (316) placé au-dessus dudit composant de transfert de fluide externe (314) en contact avec une surface de peau environnante ;
une bande de fermeture refermable (324) connectée audit champ de recouvrement (316) et ayant une position fermée la fermant et une position ouverte donnant accès audit composant de transfert de fluide externe (314) à travers celle-ci ;
une paire de champs stériles latéraux (312) situés chacun le long d'un côté respectif de ladite plaie ou incision (304) entre ledit composant de transfert de fluide externe (314) et la peau du patient ;
une source de pression (28) connectée au composant de transfert de fluide externe (314) ; et
un composant de transfert de fluide interne (310) destiné à être placé dans ladite plaie ou incision en communication fluidique avec ledit composant de transfert de fluide externe (314).

2. Pansement (302) selon la revendication 1, dans lequel ledit champ de recouvrement (316) présentant une ouverture située au-dessus dudit composant de transfert de fluide (314) ; un panneau d'accès (318) incluant ladite bande de fermeture (324) et monté sur ledit champ de recouvrement (316) avec ladite bande de fermeture (324) donnant accès de manière sélective audit composant de transfert de fluide (314) par l'ouverture dudit champ de recouvrement (316) ; ledit panneau d'accès (318) présentant une partie de périmètre (320) avec un revêtement adhésif adapté pour le fixer sur ledit champ de recouvrement (316) ; et ledit panneau d'accès (318) présentant une partie centrale sans adhésif (322) entourée par ladite partie de périmètre (320), ladite partie centrale (322) incluant ladite bande de fermeture (324).

3. Ensemble formant pansement (302) selon l'une quelconque des revendications 1 ou 2, dans lequel ladite bande de fermeture refermable (324) inclut un canal s'étendant longitudinalement (328) monté sur une première partie dudit panneau d'accès (318) et une membrure s'étendant longitudinalement (326) montée sur une seconde partie dudit panneau d'accès (318) et reçue de manière sélective dans ledit canal (328) avec ladite bande de fermeture (324) dans sa configuration fermée.
